# EUROPEAN PATENT APPLICATION

(11) **EP 1 074 831 A1**
(43) Date of publication of application: **07.02.2001**
(21) Application number: 00904090.8
(22) Date of filing: 21.02.2000
(51) Int. Cl.: G01N 21/27, G01N 33/08

(54) **METHOD AND DEVICE FOR CANDLING EGGS**

(30) Priority: 23.02.1999 JP 4487799; 28.01.2000 JP 2000020927; 28.01.2000 JP 2000020928
(71) Applicant: Kubota Corporation, Osaka-shi, Osaka, 556-8601 (JP)
(72) Inventor: IKEDA, Kazushige, Yao-shi Osaka 581-8686 (JP); IZUNO, Yuji, Yao-shi Osaka 581-8686 (JP); WATABE, Yasushi, Yao-shi Osaka 581-8686 (JP); NISHIHATA, Hironobu, Yao-shi Osaka 581-8686 (JP); NOMURA, Noriko, Yao-shi Osaka 581-8686 (JP); NARIYAMA, Keiichi, Yao-shi Osaka 581-8686 (JP); KAMADA, Akio, Yao-shi Osaka 581-8686 (JP)
(74) Representative: von Kirschbaum, Alexander, Dipl.-Ing.
(86) International application number: JP0000998
(87) International publication number: WO0050873

(57) **Abstract**

To provide an egg inspecting method capable of detecting abnormal eggs with high accuracy irrespective of the sizes of the eggs, and a device used for implementing the method.

A normalizing section divides a first signal supplied via a first band-pass filter for passing a wavelength which is absorbed to a large degree by internal abnormality by a second signal supplied via a second band-pass filter for passing a wavelength which is absorbed to a different degree depending on the color of the egg shell, divides a third signal supplied via a third band-pass filter for passing a wavelength which is absorbed to a small degree by internal abnormality by the second signal, and supplies the results to a difference calculating section. The difference calculating section calculates the difference between the results, supplies it to a judging section. The judging section compares the supplied difference with a threshold value, judges that the egg is an abnormal egg if the supplied difference is equal to or greater than the threshold value, and outputs a signal.

## Description

### TECHNICAL FIELD

The present invention relates to an egg inspecting method for non-destructively inspecting the internal condition of an egg based on transmitted light obtained by applying light to the egg, and also relates to a device for implementing the method.

### BACKGROUND ART

The eggs produced in a poultry housing include abnormal eggs such as blood-spot eggs containing small clots of blood in the egg shell and meat-spot eggs containing meat-like substances floating in the egg white or meat-like substances sticking to chalaza. Therefore, in order to ship normal eggs as food, collected eggs are non-destructively inspected to find whether they are normal or abnormal eggs.

In order to non-destructively inspect an egg, an inspector holds the egg in light emitted from a light source such as a fluorescent light and judges whether the egg is a normal egg or an abnormal egg by the eyes. However, such an inspection requires a skilled inspector, and even when a skilled inspector is employed, it is impossible for the skilled inspector to carry out the inspection for a long time. In order to solve such a problem, the following egg inspecting device has been disclosed.

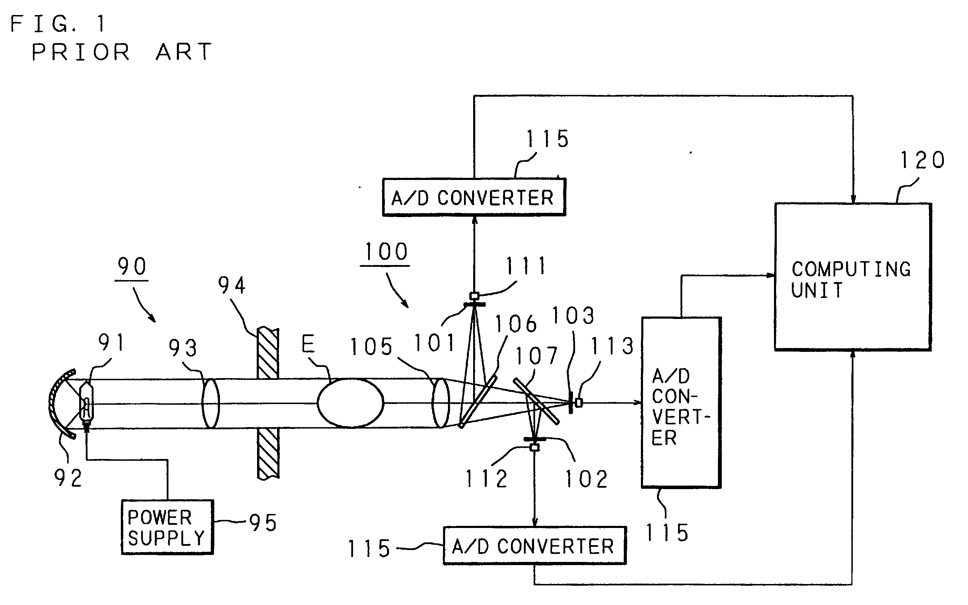

FIG. 1 is a block diagram showing the structure of a conventional egg inspecting device disclosed in Japanese Patent Publication (Application) No. 56-735(1981). In FIG. 1, numeral 90 is a light projecting unit. The light projecting unit 90 incorporates therein a halogen lamp 91 as a light source, a first optical system for guiding light emitted from the halogen lamp 91 so as to be applied to an egg E, and a power supply 95 for supplying electrical power to the halogen lamp 91, etc. The first optical system is composed of a reflector 92, a light projecting lens 93, a slit 94, etc.

The light emitted from the halogen lamp 91 by switching on the halogen lamp 91 by the power supply 95 is incident on the light projecting lens 93 directly or after being reflected by the reflector 92. The light incident on the light projecting lens 93 is made parallel light and emitted from the light projecting lens 93. The parallel light emitted by the light projecting lens 93 passes through the slit 94 and is applied to the egg E.

Meanwhile, numeral 100 in FIG. 1 is a light receiving unit. The light receiving unit 100 incorporates therein a second optical system for receiving light transmitted through the egg E and guiding the light to photoelectric converters, photo-transistors 111, 112 and 113 serving as the above-mentioned photoelectric converters, analog/digital (A/D) converters 115 connected to the photo-transistors 111, 112 and 113, respectively, and a computing unit 120 for judging whether the egg E has internal abnormality based on electrical signals from the respective A/D converters 115. The second optical system is composed of a condenser lens 105, half mirrors 106 and 107, optical filters 101, 102 and 103, etc.

The light transmitted through the egg E is incident on the condenser lens 105 of the second optical system. Two half mirrors 106 and 107 are positioned on the light emitting-side of the condenser lens 105 with a predetermined distance therebetween in the direction of the optical axis, at an angle of 45 degrees with respect to the optical axis of the condenser lens 105. The light emitted from the condenser lens 105 is condensed on a first position after being reflected by one half mirror 106; on a second position after being passed through the one the half mirror, 106, and then reflected by the other half mirror 107; and on a third position after being passed through both of the half mirrors 106 and 107. At the first to third positions, the photo-transistors 111, 112 and 113 are provided, respectively.

The optical filter 101 for passing light with a wavelength of 620 nm is disposed on the light incident side of the photo-transistor 111 provided at the first position. The optical filter 102 for passing light with a wavelength of 575 nm is disposed on the light incident side of the photo-transistor 112 provided at the second position. The optical filter 103 for passing light with a wavelength of 590 nm is disposed on the light incident side of the photo-transistor 113 provided at the third position. The photo-transistors 111 to 113 provided at the first to third positions perform photoelectric conversion of the light transmitted through the corresponding optical filters 101 to 103, and output first to third signals via the respective A/D converters 115 to the computing unit 120.

The computing unit 120 divides the given second signal by the third signal so as to normalize the second signal. If the level of the normalized signal is equal to or lower than a predetermined level, the computing unit 120 judges that the egg E is a blood-spot egg. Besides, if the given first signal is equal to or lower than a predetermined level, the computing unit 120 judges that the egg E is an addled egg. Thus, since the intensity of the transmitted light of 575 nm which is absorbed by the clots of blood is normalized by the intensity of the transmitted light of 590 nm which varies according to the color of the egg shell such as white or brown, it is possible to reduce the effect of the color of the egg shell.

In order to continuously inspect a plurality of eggs E by such a device, the above-mentioned light projecting unit 90 and light receiving unit 100 are positioned on both sides of a transporting path of the eggs E so as to face each other, and a sensor (not shown) for detecting the passage of the egg E is provided to face the transporting path on a slightly upstream side of the light projecting unit 90 and light receiving unit 100 so that a detection signal obtained by the sensor is supplied to the computing unit 120. When a predetermined time has passed after the detection signal of the egg E was supplied from the sensor, i.e., at a timing in which the egg E is present in an optical path from the light projecting unit 90 to the light receiving unit 100, the computing unit 120 reads the signals given by the respective A/D converters 115 as measured signals.

Meanwhile, a neutral-density filter (not shown) for reducing the intensity of the light emitted from the light projecting unit 90 is disposed on the light emitting-side of the slit 94 so that it can enter and exit the optical path. According to timings of sequentially detecting a plurality of eggs E by the sensor, the neutral-density filter is arranged to enter the optical path after an egg E is transported out of the optical path and to exit the optical path before the next egg E is transported into the optical path. Immediately before the egg E is transported into the optical path, in a state in which the neutral-density filter enters the optical path, the computing unit 120 reads the signals given by the A/D converters 115 as reference signals, divides the values of the above-mentioned respective measured signals by the values of the corresponding reference signals to obtain correction signals, and inspects whether the egg E has internal abnormality by the use of the respective correction signals in the manner mentioned above.

However, when the level of the normalized signal is equal to or lower than the predetermined level, a conventional egg inspecting device as described above judges that the egg is a blood-spot egg. Hence, in such a device, if a very small clot of blood is contained, the level of the normalized signal is not so low, and thus there is a possibility that such a very small clot of blood is oversight. Moreover, since the intensity of the transmitted light of 575 nm also varies according to the size of the egg (the length of the transmitted optical path) and the shade of color of the egg shell, there is a problem that the accuracy of detecting abnormal eggs is low.

Furthermore, since the light projecting unit 90 incorporates therein the power supply 95 for switching on the halogen lamp 91 and for driving a cooling system (not shown) in the unit, the overall size of the light projecting unit 90 is inevitably large. Besides, in the light receiving unit 100, the optical system (the second optical system) from the condenser lens 105 to the photo-transistors 111 to 113 is relatively large, and the degree of freedom in disposing the optical system is limited. Consequently, the degree of freedom in disposing the light projecting unit 90 and light receiving unit 100 on both sides of the transporting path of the eggs E is limited, thereby making it particularly difficult to introduce the egg inspecting device into an existing transporting system.

The present invention has been made to solve the above-mentioned problems, and an object of the present invention is to provide an egg inspecting method capable of detecting abnormal eggs with high accuracy irrespective of the size of the egg and the shade of the egg shell by measuring the intensity of a first wavelength which is absorbed to a large degree by an egg having internal abnormality, the intensity of a second wavelength which is absorbed to a different degree depending on the type of the egg shell and the intensity of a third wavelength which is absorbed to a small degree by an egg having internal abnormality respectively by the use of transmitted light through an egg; normalizing each of the measured intensities of the third wavelength and first wavelength by the intensity of the second wavelength; calculating the difference between the normalized intensity of the first wavelength and the normalized intensity of the third wavelength; and judging whether the egg has internal abnormality based on the calculated difference, and to provide a device used for implementing this method.

Another object of the present invention is to provide an egg inspecting device which can be introduced into an egg transporting system without being restricted by the sizes of a light projecting unit and light receiving unit and has a high degree of freedom in disposing both of the units by using optical fibers to form part or all of first and second optical systems in an egg inspecting device configured such that light from a light source is guided by the first optical system so as to be applied to an egg, transmitted light from the egg is guided to a photoelectric converter by the second optical system and the inside of the egg is inspected based on the magnitude of an electrical signal resulting from the photoelectric conversion.

A further object of the present invention is to provide an egg inspecting device capable of increasing the ratio of light applied to an egg or reducing external light and stray light incident on a light receiver, or preventing a deviation of the wavelength of light passing through an optical filter, by improving the structure of the optical system.

### SUMMARY OF THE INVENTION

An egg inspecting method according to the first aspect, which is an egg inspecting method comprising applying light including predetermined wavelengths to an egg from a light source so as to obtain transmitted light; measuring an intensity of a first wavelength which is absorbed to a large degree by internal abnormality of the egg and an intensity of a second wavelength which is absorbed to a different degree depending on a type of a shell of the egg respectively by using the transmitted light; and detecting internal abnormality of the egg based on the measured intensities of the first and second wavelengths, is characterized by measuring an intensity of a third wavelength which is absorbed to a small degree by the internal abnormality by using the transmitted light; normalizing the measured intensities of the third wavelength and first wavelength respectively by the intensity of the second wavelength; calculating a difference between the normalized intensity of the first wavelength and the normalized intensity of the third wavelength; and judging whether the egg has internal abnormality based on the calculated difference.

An egg inspecting device used for implementing this method, which is an egg inspecting device comprising a measuring section for measuring an intensity of a first wavelength which is included in transmitted light obtained by applying light including predetermined wavelengths to an egg and absorbed to a large degree by internal abnormality of the egg; a measuring section for measuring an intensity of a second wavelength which is absorbed to a different degree depending on a type of a shell of the egg by using the transmitted light; and a detector for detecting internal abnormality of the egg based on the measured intensities of the first and second wavelengths, is characterized by comprising a measuring section for measuring an intensity of a third wavelength which is absorbed to a small degree by the internal abnormality by using the transmitted light, and characterized in that the detector includes a normalizing section for normalizing the third intensity and the first intensity respectively by the second intensity, and a difference calculating section for calculating a difference between the normalized first intensity and third intensity.

Light including predetermined wavelengths is applied to an egg from a light source such as a lamp or natural light so as to obtain transmitted light, and the transmitted light is used to measure the intensity of the first wavelength which is absorbed to a large degree by internal abnormality of the egg, the intensity of the second wavelength which is absorbed to a different degree depending on the type of the egg shell and the intensity of the third wavelength which is absorbed to a small degree by the internal abnormality.

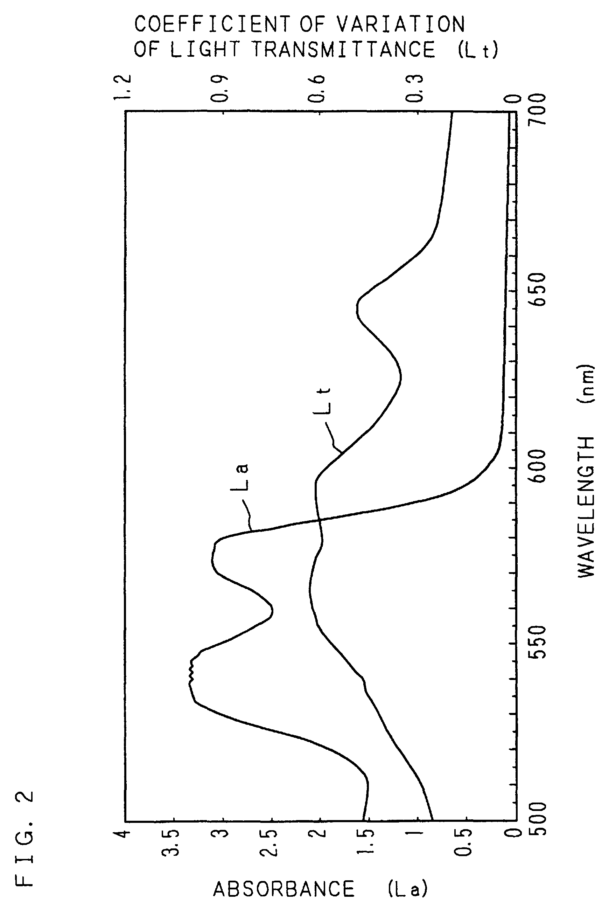

FIG. 2 is a graph showing the absorption spectrum of blood and the coefficient of variation of transmittance of a plurality of eggs, wherein the left side vertical axis indicates the absorbance, the right side vertical axis shows the coefficient of variation of the light transmittance (standard deviation/mean value), and the horizontal axis represents the wavelength. Further, in FIG. 2, the La line indicates the absorption spectrum of blood, and the Lt line shows the coefficient of variation of the light transmittance of a plurality of eggs having different shades in color and different sizes.

As is apparent from the La line shown in FIG. 2, the absorbance of blood has the maximum value in the vicinity of 540 nm and the vicinity of 575 nm, and the minimum value in the vicinity of 560 nm. Light with wavelengths longer than 610 nm is scarcely absorbed by blood. Moreover, it is apparent from the Lt line that the coefficient of variation of the light transmittance of a plurality of eggs having different shades in color and sizes is large at 645 nm which is scarcely absorbed by blood.

Therefore, when the internal abnormality is clots of blood, the above-mentioned second wavelength is in the vicinity of 645 nm, and the third wavelength is in the vicinity of 560 nm. Besides, the first wavelength is in the vicinity of 575 nm which has high detection sensitivity.

Further, the intensities of the first to third wavelengths may be measured by dividing the transmitted light to measure each intensity of the wavelengths, or by making the transmitted light incident on an optical filter for passing each wavelength and by measuring the intensity of the light passed through the optical filter.

The intensities of the third wavelength and first wavelength thus measured are normalized by, for example, dividing the intensities of the third wavelength and first wavelength by the intensity of the second wavelength, and the difference between the normalized intensity of the first wavelength and the normalized intensity of the third wavelength is calculated. If the calculated difference is smaller than a preset value, the inspected egg is judged to be an abnormal egg.

Hence, since the intensity of the first wavelength which is absorbed to a large degree by internal abnormality and the intensity of the third wavelength which is absorbed to a small degree by internal abnormality are normalized by the intensity of the second wavelength which is absorbed to a different degree depending on the size of the egg and the shade of color of the egg shell, it is possible to reduce the effects of the size of the egg and the shade of color of the egg shell. Moreover, since the difference between the normalized intensity of the first wavelength and the normalized intensity of the third wavelength is calculated, the effects of the size of the egg and the shade of color of the egg shell are further reduced, and the presence of the internal abnormality can be accurately judged. It is therefore possible to detect very small internal abnormality and detect abnormal eggs with high accuracy, irrespective of the shade of color of the egg shell and the size of the egg.

An egg inspecting device according to the second aspect, which is an egg inspecting device comprising a light source; a first optical system for guiding the light from the light source so as to be applied to an egg; a second optical system for guiding the transmitted light from the egg; a photoelectric converter for performing photoelectric conversion of the light guided by the second optical system; and an inspecting section for inspecting the inside of the egg based on the magnitude of an electrical signal obtained by the photoelectric converter, is characterized in that the first optical system and second optical system are partially or entirely formed by optical fibers.

Since optical fibers are used to form part or all of the first and second optical systems in the egg inspecting device that guides light from the light source such as a halogen lamp by the first optical system so as to be applied to an egg, guides the light applied to and transmitted through the egg to the photoelectric converter by the second optical system, performs photoelectric conversion of the guided light in the photoelectric converter and inspects the inside of the egg based on the magnitude of a resultant electrical signal by the inspecting section, this egg inspecting device can be readily introduced into an egg transporting system without being restricted by the size of a light projecting unit including the light source and first optical system and the size of a light receiving unit including the second optical system, photoelectric converter and inspecting section, thereby increasing the degree of freedom in disposing both of the units.

Another egg inspecting device of the second aspect is characterized in that a light emitting end of the first optical system to the egg and a light incident end of the second optical system from the egg are disposed in another housing separated from a housing storing the light source, photoelectric converter and inspecting section.

In this egg inspecting device, since the light emitting end of the first optical system to the egg and the light incident end of the second optical system from the egg are disposed in another housing separated from the housing storing the light source, photoelectric converter and inspecting section, it is possible to have the light emitting end and light incident end separate from most parts of the device by optical fibers, thereby further improving the degree of freedom in disposing the device. In addition, since the above-mentioned light emitting end and light incident end are disposed in a single housing, it is possible to eliminate the necessity of aligning the optical axes of these light emitting end and light incident end.

Still another egg inspecting device of the second aspect is characterized in that the light emitting end of the first optical system to the egg is positioned in a second housing which is separated from a first housing storing the light source, and the light incident end of the second optical system from the egg is positioned in a fourth housing which is separated from a third housing storing the photoelectric converter and inspecting section.

In this egg inspecting device, the light emitting end of the first optical system to the egg is positioned in the second housing separated from the first housing storing the light source, and the light incident end of the second optical system from the egg is positioned in the fourth housing separated from the third housing storing the photoelectric converter and inspecting section. Hence, the light projecting unit and the light receiving unit can be included separately, thereby further improving the degree of freedom in disposing the device. Moreover, the light emitting end of the first optical system is positioned in a housing different from a housing of the main body section of the light projecting unit, and the light incident end of the second optical system is positioned in a housing different from a housing of the main body section of the light receiving unit. Therefore, the degree of freedom in disposing the device is further improved.

However, if the light emitting end is an optical fiber with a relatively large numerical aperture, there is a possibility that part of the emitted light is not applied to the egg and the utilization factor is lowered. Similarly, if the light incident end is an optical fiber with a relatively large numerical aperture, there is a possibility that external light and stray light which is not applied to the egg are received in addition to the light transmitted through the egg. Furthermore, since light incident on the light incident end formed by an optical fiber from an oblique direction with respect to the center axis of the optical fiber is emitted from the other end of the optical fiber at the same angle as the incident angle, this light is obliquely incident on a band-pass filter. Therefore, if the band-pass filter is an interference filter, the optical path difference of light in the interference filter is smaller compared with that of light incident on the interference filter at a right angle, and thus there is a possibility that the characteristic of the interference filter is shifted to shorter wavelength side.

Then, an egg inspecting device according to the third aspect, which is an egg inspecting device comprising a light source; a first light guiding body for guiding light emitted by the light source; a projector for emitting the light guided by the first light guiding body to an egg; a light receiver on which the transmitted light through the egg is incident; a second light guiding body for guiding the transmitted light incident on the light receiver; and a detector for detecting internal abnormality of the egg based on the intensity of the transmitted light guided by the second light guiding body, is characterized in that the projector includes a condenser lens for condensing the light emitted from the first light guiding body.

In this egg inspecting device, the light emitted by the light source is guided to the projector by an optical fiber functioning as the first light guiding body. The projector is formed by disposing the condenser lens on the light emitting-side end portion of the first light guiding body, and the light emitted from the light emitting-side end portion of the first light guiding body is converged into a beam of a suitable diameter by the condenser lens and applied to the egg from the projector. Hence, the ratio of the light applied to the egg to the light emitted by the projector is improved irrespective of the numerical aperture of the first light guiding body. Therefore, if the quantity of light of the light source is uniform, the quantity of light transmitted through an egg is increased, while the stray light is reduced as compared with a conventional device, thereby improving the egg inspection accuracy.

Another egg inspecting device of the third aspect, which is an egg inspecting device comprising a light source; a first light guiding body for guiding light emitted by the light source; a projector for emitting the light guided by the first light guiding body to an egg; a light receiver on which the transmitted light through the egg is incident; a second light guiding body for guiding the transmitted light incident on the light receiver; and a detector for detecting internal abnormality of the egg based on the intensity of the transmitted light guided by the second light guiding body, is characterized in that the projector and/or the light receiver include(s) a light blocking cylinder, and the first light guiding body and/or the second light guiding body are/is connected to the cylinder so that a light emitting-side end portion of the first light guiding body and/or a light incident-side end portion of the second light guiding body are/is positioned in the cylinder.

In this egg inspecting device, if the light emitting-side end portion of the first light guiding body is positioned in the light blocking cylinder disposed in the projector, even when the numerical aperture of the first light guiding body is relatively large, the divergence of the emitted light from the projector is prevented by the cylinder, thereby reducing stray light which is not applied to the egg. Besides, if the light incident-side end portion of the second light guiding body is positioned in the light blocking cylinder disposed in the light receiver, the transmitted light through the egg which is substantially parallel to the axial direction of the cylinder enters the cylinder, while external light and stray light which travel in a direction across the axial direction of the cylinder can hardly enter the cylinder. Therefore, even if the numerical aperture of the second light guiding body is relatively large, it is possible to prevent the external light and stray light from falling on the second light guiding body.

Still another egg inspecting device of the third aspect, which is an egg inspecting device comprising a light source; a first light guiding body for guiding light emitted by the light source; a projector for emitting the light guided by the first light guiding body to an egg; a light receiver on which the transmitted light through the egg is incident; a second light guiding body for guiding the transmitted light incident on the light receiver; and a detector for detecting internal abnormality of the egg based on the intensity of the transmitted light guided by the second light guiding body, is characterized in that an interference filter for passing light in a predetermined wavelength band is disposed to face a light emitting-side end portion of the second light guiding body, and a collimate lens is installed between the light emitting-side end portion of the second light guiding body and the interference filter.

In this egg inspecting device, the light guided to the detector by the second light guiding body is emitted from the light emitting-side end portion of the second light guiding body to the interference filter, and light in the predetermined wavelength band passes through the interference filter. In this case, since the collimate lens is installed between the light emitting-side end portion of the second light guiding body and the interference filter, light which has been made parallel light by the collimate lens is incident on the interference filter. It is therefore possible to prevent a deviation of the wavelength of light passing through the interference filter.

Yet another egg inspecting device of the third aspect is characterized by comprising a third light guiding body for guiding light emitted by the light source to the detector; another interference filter, disposed to face a light emitting-side end portion of the third light guiding body, for passing light in the same wavelength band as the wavelength band of the above-mentioned interference filter; and another collimate lens installed between the light emitting-side end portion of the third light guiding body and the another interference filter.

In this egg inspecting device, in order to obtain a reference as the value of the quantity of light of the light source used for obtaining the absorbance from the light transmitted through an egg, the light emitted by the light source is guided to the detector by the third light guiding body. Another interference filter for passing light in the same wavelength band as the wavelength band of the interference filter provided on the light emitting-side end portion of the second light guiding body is disposed on the light emitting-side end portion of the third light guiding body, and another collimate lens is installed between the another interference filter and the light emitting-side end portion of the third light guiding body. Hence, similarly to the above, it is possible to prevent a deviation of the wavelength of light passing through the another interference filter, thereby providing a highly reliable reference.

Further, by mutually combining the above-explained first through third aspects, it is also possible to provide an egg inspecting device capable of inspecting eggs with higher accuracy and reliability.

### BRIEF DESCRIPTION OF THE DRAWINGS

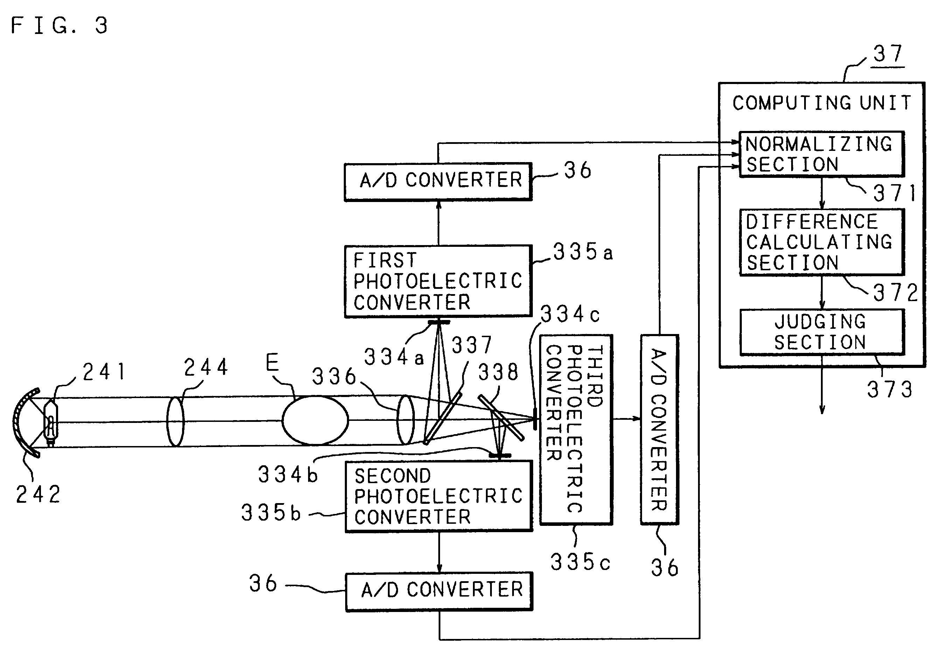

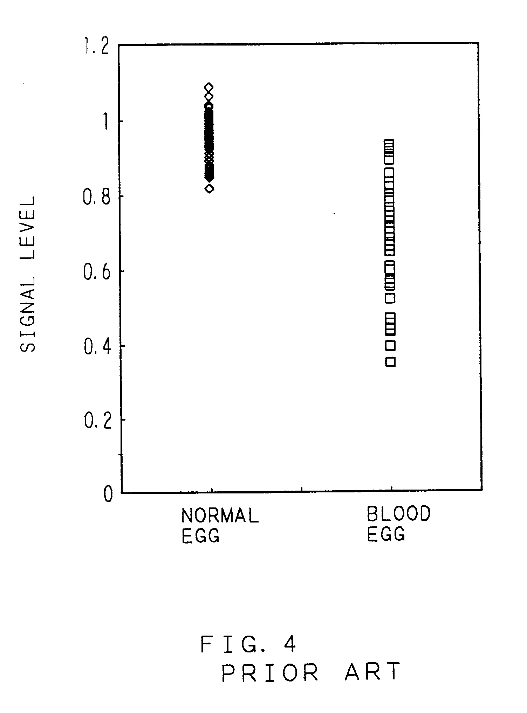

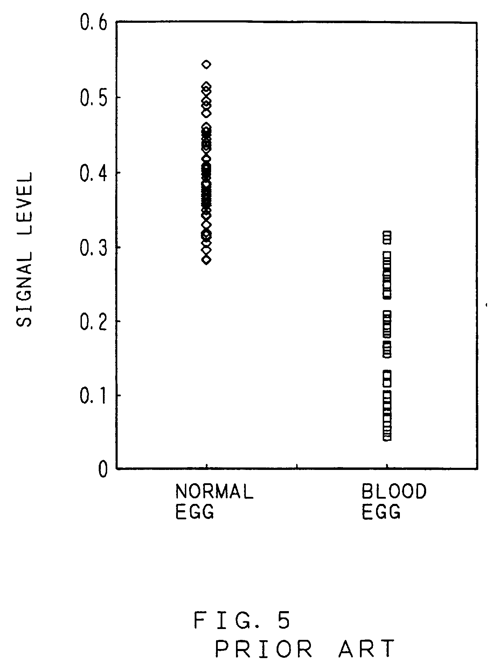

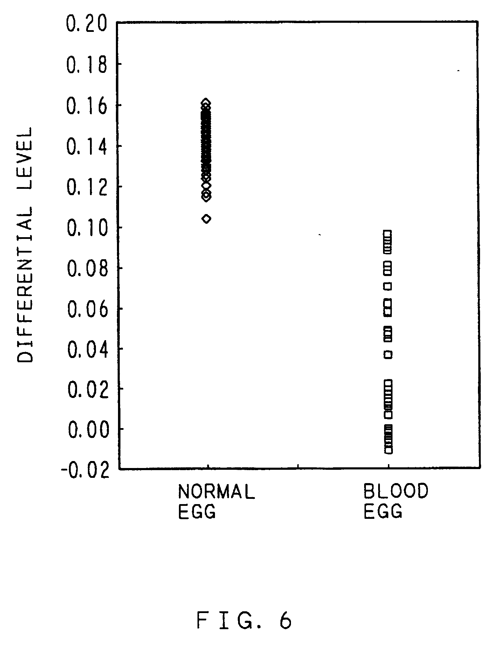

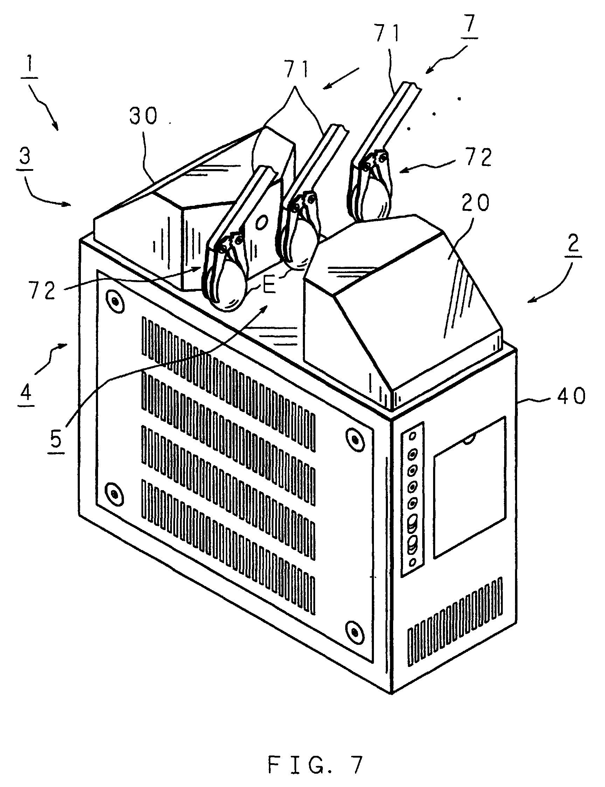

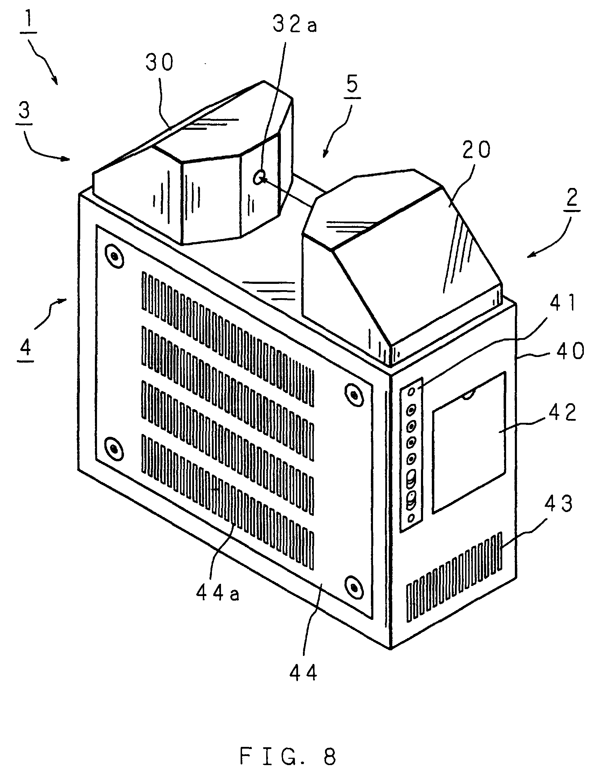

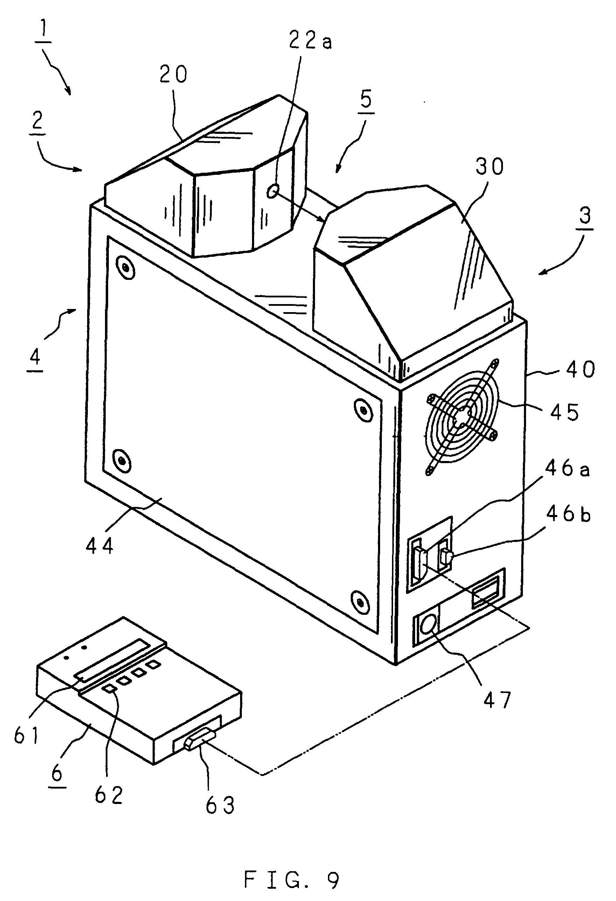

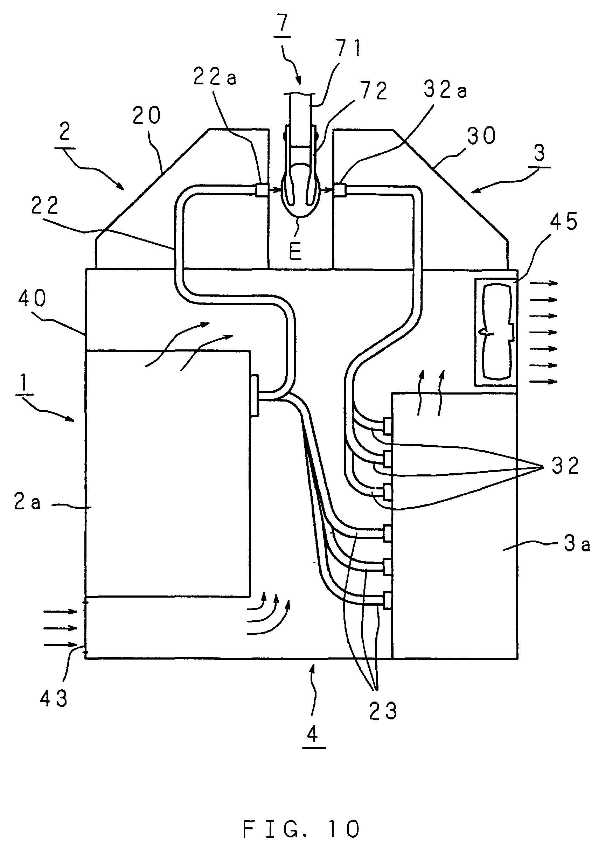

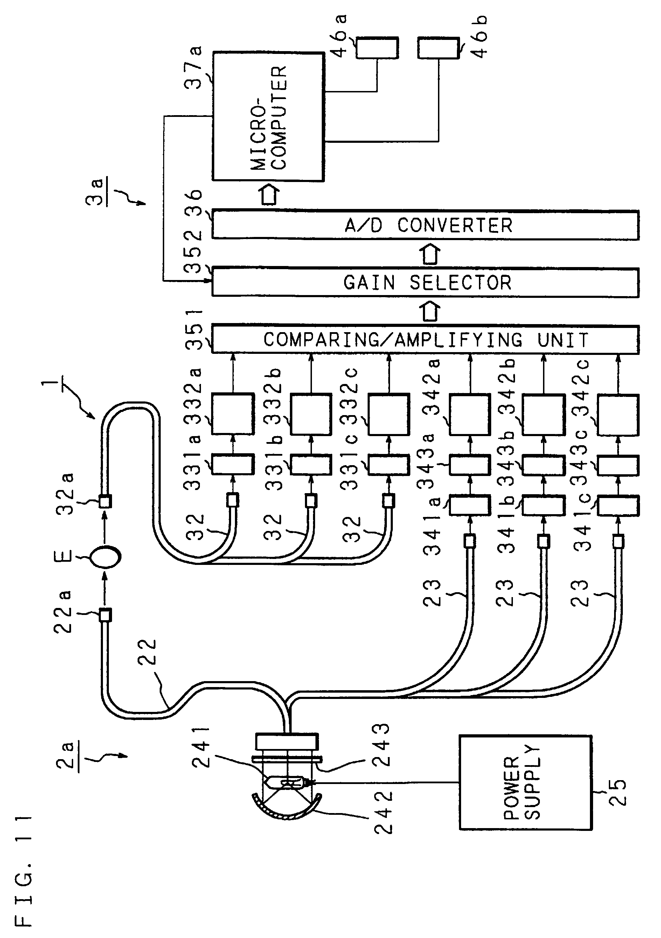

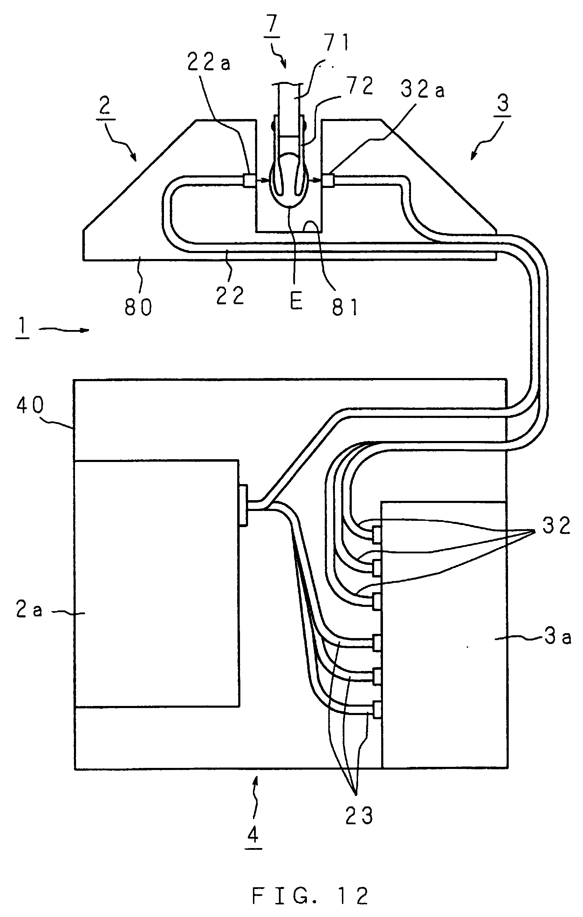

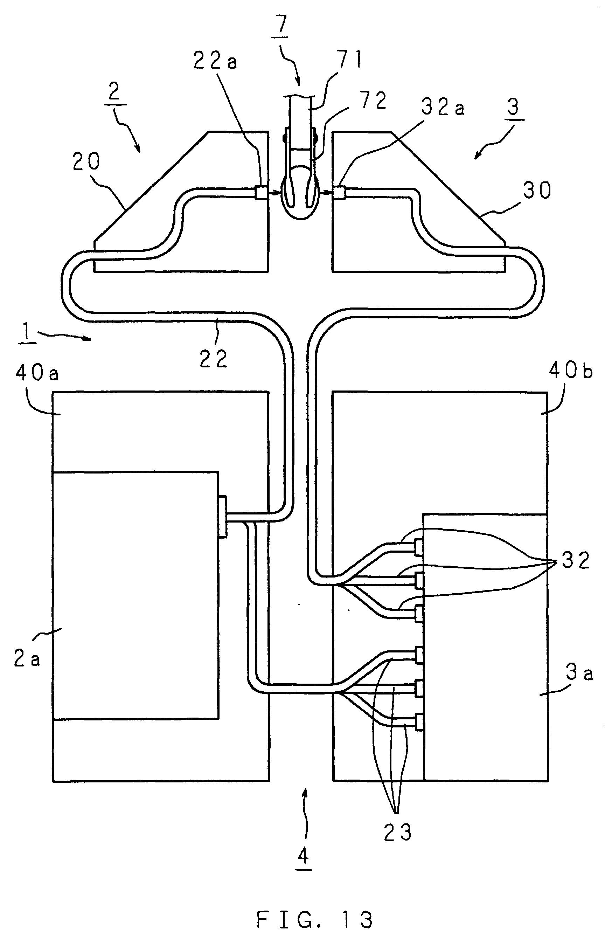

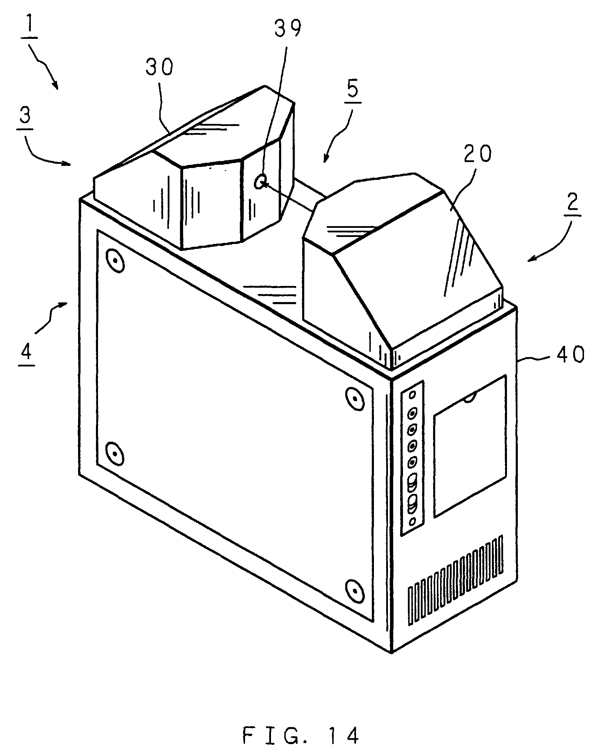

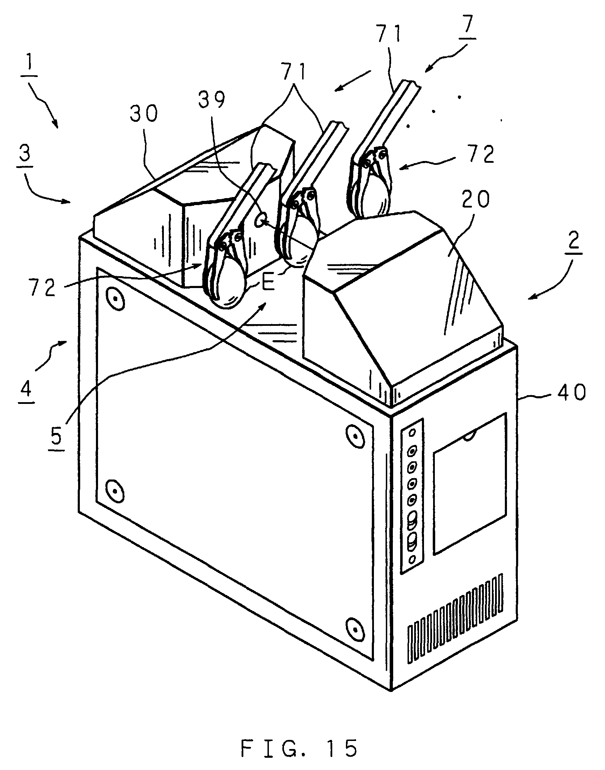

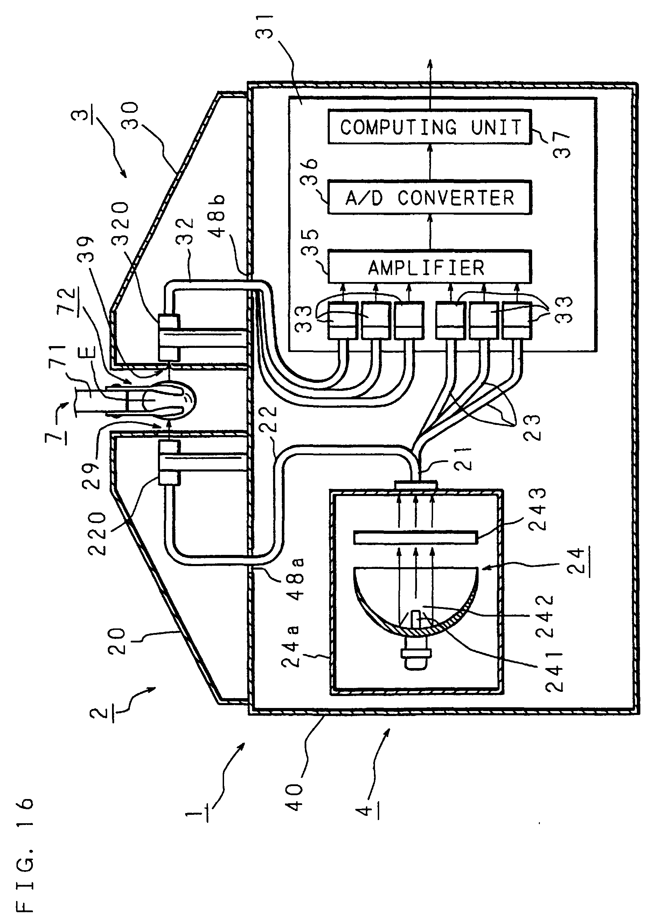

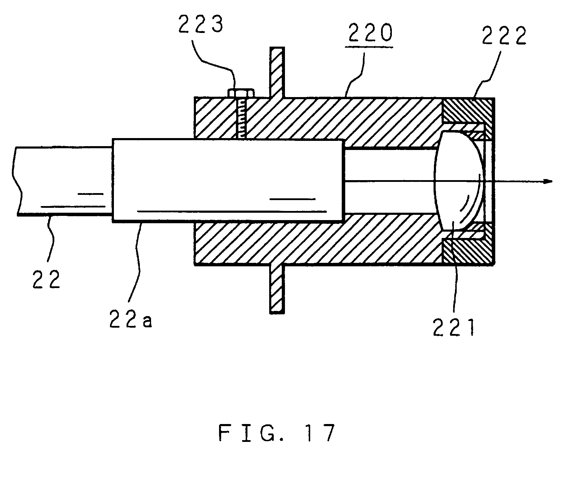

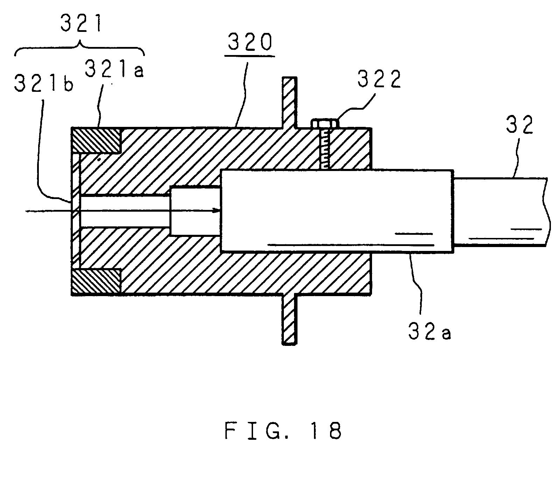

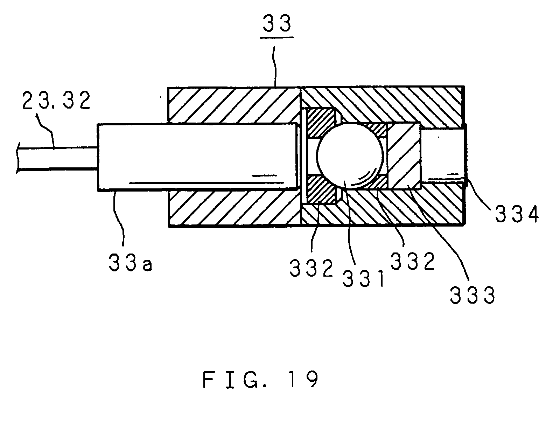

FIG. 1 is a block diagram showing the structure of an egg inspecting device disclosed in the Japanese Patent Publication (Application) No. 56-735(1981); FIG. 2 is a graph showing the absorption spectrum of blood and the coefficient of variation of transmittance of a plurality of eggs; FIG. 3 is a block diagram showing the structure of a first embodiment of an egg inspecting device according to the present invention; FIG. 4 is a graph showing the results of inspecting a normal egg and an abnormal egg containing clots of blood by the conventional egg inspecting device disclosed in the Japanese Patent Publication (Application) No. 56-735(1981); FIG. 5 is a graph showing the results of inspecting a normal egg and an abnormal egg containing clots of blood by the above-mentioned conventional egg inspecting device in which the transmission wavelength of an optical filter for obtaining a second signal is varied; FIG. 6 is a graph showing the results of inspecting a normal egg and an abnormal egg containing clots of blood by the egg inspecting device of the first embodiment according to the present invention; FIG. 7 is a perspective view showing a second embodiment of an egg inspecting device according to the present invention together with the essential sections of a transporting device of eggs; FIG. 8 is a perspective view of only the egg inspecting device of FIG. 7 seen from the front upper side; FIG. 9 is a perspective view of only the egg inspecting device of FIG. 7 seen from the rear upper side; FIG. 10 is a side cross section showing the internal structure of the second embodiment of the egg inspecting device according to the present invention from the right side; FIG. 11 is a block diagram showing the structure of the second embodiment of the egg inspecting device according to the present invention; FIG. 12 is a side cross section showing the entire structure of a third embodiment of an egg inspecting device according to the present invention from the right side; FIG. 13 is a side cross section showing the entire structure of a fourth embodiment of an egg inspecting device according to the present invention from the right side; FIG. 14 is an external perspective view showing a fifth embodiment of an egg inspecting device according to the present invention; FIG. 15 is a perspective view showing a state in which an egg is being inspected by the egg inspecting device shown in FIG. 14; FIG. 16 is a block diagram showing the structure of a main body of the egg inspecting device shown in FIG. 15; FIG. 17 is a side cross section of a light projecting cylinder and the vicinity of the light emitting end of a light projecting optical fiber shown in FIG. 16; FIG. 18 is a side cross section of a light receiving cylinder and the vicinity of the light incident end of a light receiving optical fiber shown in FIG. 16; and FIG. 19 is a side cross section of a sensor block shown in FIG. 16 and a light emitting end connected to the sensor block.

### PREFERRED EMBODIMENTS OF THE INVENTION

The following description will specifically explain the embodiments of the present invention with reference to the drawings.

### (First Embodiment)

FIG. 3 is a block diagram showing the structure of the first embodiment of an egg inspecting device according to the present invention. In FIG. 3, numeral 241 is a halogen lamp as a light source for emitting light including wavelengths ranging from 500 nm to 700 nm. A reflector 242 is positioned so as to be separated from the halogen lamp 241 by a predetermined distance, so that the light emitted from the halogen lamp 241 and the light reflected by the reflector 242 are incident on a first lens 244. The first lens 244 makes the incident light parallel light having substantially the same cross-sectional area as an egg E, and emits the parallel light to a second lens 336 disposed to face the first lens 244.

The egg E is positioned between the first lens 244 and the second lens 336, and the light transmitted through the egg E is condensed by the second lens 336. On an outgoing optical path from the second lens 336, a first half mirror 337 and a second half mirror 338 are positioned with a suitable distance therebetween, at predetermined angles with respect to the optical path. The light reflected by the first half mirror 337 is incident on a first photoelectric converter 335a via a first band-pass filter 334a for passing, for example, light with wavelengths in the vicinity of 575 nm which is absorbed to a large degree by clots of blood.

The light passed through the first half mirror 337 and reflected by the second half mirror 338 is incident on a second photoelectric converter 335b via a second band-pass filter 334b for passing light with wavelengths in the vicinity of 645 nm which is absorbed to a different degree depending on the color of the egg shell. The light passed through the first and second half mirrors 337 and 338 is incident on a third photoelectric converter 335c via a third band-pass filter 334c for passing light with wavelengths in the vicinity of 560 nm which is absorbed to a small degree by clots of blood.

Further, the first and second half mirrors 337 and 338 are arranged such that the intensity of light reflected by the first half mirror 337, the intensity of light passed through the first half mirror 337 and reflected by the second half mirror 338 and the intensity of light passed through the first and second half mirrors 337 and 378 have the same value respectively.

The first to third photoelectric converters 335a to 335c convert the incident light into electrical signals according to the intensity of the light, and supply the resultant electrical signals to a normalizing section 371 of a computing unit 37 via analog/digital (A/D) converters 36, respectively. The normalizing section 371 performs normalizing processing for removing the effect of the egg shell from the first and third signals by dividing the signal (first signal) supplied from the first photoelectric converter 335a by the signal (second signal) supplied from the second photoelectric converter 335b and dividing the signal (third signal) supplied from the third photoelectric converter 335c by the second signal, and supplies the normalized first and third signals to a difference calculating section 372.

The difference calculating section 372 calculates the difference between the first and third signals by subtracting the first signal from the third signal, and supplies the difference to a judging section 373. The judging section 373 has a preset threshold value for judging whether the egg E is good or bad, and compares the supplied difference with the threshold value. If the supplied difference is equal to or smaller than the threshold value, the judging section 373 judges that the egg E is an abnormal egg and outputs a signal.

Further, in this embodiment, although the egg E is inspected by the use of the first signal obtained through the first band-pass filter 334a for passing light with wavelengths in the vicinity of 575 nm which is absorbed to a large degree by clots of blood, the present invention is not necessarily limited to such an inspection. It is possible to inspect the egg E by the use of a signal which is obtained through a band-pass filter for passing light with wavelengths corresponding to other internal abnormality, such as meat-like substances or the turbidity of egg white, instead of the first signal or in addition to the first signal. In this case, the obtained signal is normalized by the use of the above-mentioned second signal, and the difference between the normalized signal and the normalized third signal or a normalized signal obtained by performing photoelectric conversion of light with other wavelength which is absorbed to a small degree by this internal abnormality is calculated. In this manner, it is possible to detect very small internal abnormality with high accuracy, irrespective of the types of the egg shell and the sizes of the eggs E.

Next, the following description will explain the results of comparison tests.

FIG. 4 is a graph showing the results of inspecting a normal egg and an abnormal egg (blood egg) containing clots of blood by a conventional egg inspecting device disclosed in the Japanese Patent Publication (Application) No. 56-735(1981), with a vertical axis representing the signal level of the first signal normalized by the second signal. In this case, the first signal was obtained by an optical filter for passing light of 575 nm, and the second signal was obtained by an optical filter for passing light of 590 nm.

Moreover, FIG. 5 is a graph showing the results of inspecting a normal egg and an abnormal egg (blood egg) containing clots of blood by the above-mentioned conventional egg inspecting device in which the wavelength passed through the optical filter for obtaining the second signal was varied. In this case, the first signal was obtained by an optical filter for passing light of 575 nm and the second signal was obtained by an optical filter for passing light of 645 nm.

In contrast, FIG. 6 is a graph showing the results of inspecting a normal egg and an abnormal egg containing clots of blood by an egg inspecting device of this embodiment, with the vertical axis representing the differential level between the first signal normalized by the second signal and the third signal normalized by the second signal. In the egg inspecting device of this embodiment, the first signal was obtained by an optical filter for passing light of 575 nm, the second signal was obtained by an optical filter for passing light of 645 nm, and the third signal was obtained by an optical filter for passing light of 560 nm.

As is apparent from FIGS. 4 and 5, in the conventional device, a part of the result of inspecting the normal egg overlaps a part of the result of inspecting the abnormal egg, irrespective of the wavelength passed through the optical filter for obtaining the second signal, and therefore a detection error or oversight of the abnormal egg occurs. On the other hand, as is apparent from FIG. 6, in the egg inspecting device of this embodiment, the result of inspecting the normal egg and the result of inspecting the abnormal egg do not overlap each other. For instance, by setting a threshold value at 0.10, it is possible to detect abnormal eggs with high accuracy.

### (Second Embodiment)

FIG. 7 is a perspective view showing the second embodiment of an egg inspecting device according to the present invention together with the essential sections of a transporting device of eggs E. FIG. 8 is a perspective view of only the egg inspecting device of FIG. 7 seen from the front upper side. FIG. 9 is a perspective view of only the egg inspecting device of FIG. 7 seen from the rear upper side.

In FIG. 7, numeral 7 is a transporting device of eggs E. In the transporting device 7, a plurality of arms 71 hanged from a plurality of supporting members (not shown) are arranged in an array at predetermined intervals. The lower end of each of the arms 71 is provided with a nip section 72 formed by attaching a plurality of fingers so that the fingers are freely movable in forward and backward directions and the egg E is held by each nip section 72.

Each of the above-mentioned supporting members is supported by a horizontally disposed rail (not shown) so that the respective supporting members can freely slide in forward and backward directions. By moving the respective supporting members in one direction with a drive device (not shown), the eggs E held by the respective nip sections 72 are transported in the direction indicated by an arrow. The egg inspecting device 1 of this embodiment is configured such that the light projecting end section 2 for applying light to the egg E and the light receiving end section 3 on which the light transmitted through the egg E is incident are positioned to face each other so that they face the transporting region of the eggs E.

It is clear from FIGS. 8 and 9 that the egg inspecting device 1 of this embodiment includes the above-described light projecting end section 2, light receiving end section 3 and a later-described egg inspecting device main body 4. On the upper surface of a rectangular parallelepiped main body housing 40 as a housing of the egg inspecting device main body 4, a light projecting end section cover 20 that is a box-like housing of the light projecting end section 2 and a light receiving end section cover 30 that is a box-like housing of the light receiving end section 3 are disposed with a predetermined distance therebetween in a longitudinal direction of the main body housing 40. The mutually facing side faces of the light projecting end section cover 20 and light receiving end section cover 30 form a transporting path 5.

When seen from above, each of the light projecting end section cover 20 and light receiving end section cover 30 has a hexagonal shape formed by changing one side of a rectangle to a trapezoidal shape. Besides, when seen from side, each of the light projecting end section cover 20 and light receiving end section cover 30 has a pentagonal shape formed by cutting away the upper side and a side opposite to the above-mentioned one side to form a bevel. The light projecting end section cover 20 and light receiving end section cover 30 are arranged so that their side faces corresponding to the above-mentioned one side face each other.

More specifically, the center portions of the mutually facing surfaces of the light projecting end section cover 20 and light receiving end section cover 30 are parallel with each other, and the portions on both sides of the center portions are respectively tapered. Therefore, when seen from above, the transporting path 5 has a so-called Japanese hand drum shape (bi-concave shape) with a narrower width at the center portion and a gradually increasing width toward both of the end portions.

Regarding the main body housing 40, in FIG. 8, the right side (the light projecting end section 2 side) serves as its front face. The front face of the main body housing 40 is provided with a console panel 41 including lamps for indicating the operation state of the egg inspecting device 1 and a switch for operating the main power supply, a door 42 which covers an opening for the replacement of a later-described halogen lamp so as to freely open and close the opening, and a later-described air inlet 43 for cooling the inside.

Moreover, a side face of the main body housing 40, which corresponds to a downstream side of the transporting direction of the egg E, is covered with a detachable panel 44 in the shape of a rectangular plate for the maintenance of the inside. An air inlet 44a is formed over the entire surface of this detachable panel 44.

Meanwhile, as shown in FIG. 9, the rear face located on the light receiving end section 3 side is provided with a cooling fan 45, two kinds of connectors 46a and 46b, and a terminal 47 for the supply of power from outside. A remote controller 6 incorporating therein a microcomputer (not shown) for monitoring and operating the egg inspecting device 1 is connected to one connector 46a, with a suitable connecting cable. This remote controller 6 includes at least a liquid crystal panel 61 for monitoring and displaying operational set values, an operating button 62 for entering settings, and a connector 63 for connecting the remote controller 6 to the connector 46a.

Moreover, a side face of the main body housing 40 that corresponds to the upstream side of the transporting direction of the egg E is also covered with the detachable panel 44 for the maintenance of the inside. However, the air inlet 44a is not formed in the detachable panel 44 provided on this side. Therefore, even if the egg E falls down from the nip section 72 of the transporting device 7 for some reason and crashes against the detachable panel 44 during the transport, the content of the egg E does not enter the inside of the main body housing 40 and thus causes no trouble. However, instead of the opening configuration of the air inlet 44a disclosed in this embodiment, if, for example, an overhang for preventing the entry of the content of the egg E from an upper direction is provided, the air inlet 44a may be formed on both of the right and left sides of the main body housing 40.

FIG. 10 is a side cross section showing the internal structure of the second embodiment of the egg inspecting device 1 according to the present invention from the right side. The egg inspecting device 1 of this embodiment includes mainly a light projecting unit 2a and a light receiving unit 3a in its main body housing 40. The light projecting unit 2a has a box-like shape on the whole and is tightly fastened to the inner surface of the front face of the main body housing 40 at a position above the above-mentioned air inlet 43 formed at a lower position in the front face of the main body housing 40 so as not to close the air inlet 43. Meanwhile, like the light projecting unit 2a, the light receiving unit 3a has a box-like shape on the whole, and is tightly fastened to the inner surface of the rear face of the main body housing 40 at a position below the above-mentioned cooling fan 45 attached at an upper position on the rear face of the main body housing 40 so as not to close the cooling fan 45.

With such an arrangement of the light projecting unit 2a and light receiving unit 3a, a flow of air is produced by driving the cooling fan 45 to rotate, and external air introduced into the main body housing 40 from the air inlet 43 circulates around the light projecting unit 2a to dissipate heat generated mainly by a later-described light source in the light projecting unit 2a. This air also circulates around the light receiving unit 3a and is discharged in a forced manner from the cooling fan 45 while dissipating heat generated mainly by a later-described electric circuit in the light receiving unit 3a. In short, the external air with a relatively low temperature is introduced from a lower position of the main body housing 40, particularly a lower position of the light projecting unit 2a that generates a large amount of heat, flows in the inside of the main body housing 40 and is discharged from the cooling fan 45 provided at a higher position of the main body housing 40, thereby achieving a high cooling efficiency. Besides, this cooling effect is enhanced by the air inlet 44a formed in the detachable panel 44 as described above.

By the way, a light projecting optical fiber 22 whose one end is connected to the light source incorporated in the light projecting unit 2a and three reference optical fibers 23 whose one end is connected to the light source are led from the light projecting unit 2a. The other end of the light projecting optical fiber 22 passes through the upper face of the main body housing 40 and is led into the light projecting end section cover 20, and its light projecting end 22a serving as a light emitting end with respect to the egg E is fastened to a hole portion formed in a side face of the light projecting end section cover 20, which faces the transporting path 5. Besides, the other end of each of the reference optical fibers 23 is individually led into the light receiving unit 3a.

On the other hand, three light receiving optical fibers 32 are led from the light receiving unit 3a. These light receiving optical fibers 32 pass through the upper face of the main body housing 40, are led into the light receiving end section cover 30, united into one line at their light receiving end 32a serving as a light incident end on which the light from the egg E is incident, and fastened to a hole portion formed in a side face of the light receiving end section cover 30, which faces the transporting path 5.

FIG. 11 is a block diagram showing the structure of the first embodiment of the egg inspecting device 1 according to the present invention. In the egg inspecting device 1 of the present invention, the light projecting unit 2a includes the halogen lamp 241 as a light source, reflector 242, a heat ray cut filter 243 for cutting a heat ray (infrared ray), a power supply 25, etc. When the halogen lamp 241 is switched on by the power supply 25, light emitted from the halogen lamp 241 is incident on the heat ray cut filter 243 directly or after being reflected by the reflector 242. The heat components are removed by the heat ray cut filter 243, and the resultant light is incident on the light projecting optical fiber 22. This incident light is guided to the above-mentioned light projecting end 22a by the light projecting optical fiber 22, and applied from the light projecting end 22a to the egg E positioned at the center of the transporting path 5. The light transmitted through the egg E is incident on the light receiving end 32a of the above-mentioned light receiving optical fibers 32, and guided into the light receiving unit 3a by these light receiving optical fibers 32.

Moreover, the light from which the heat ray components have been removed by the heat ray cut filter 243 is divided and incident on the three reference optical fibers 23, and then guided into the light receiving unit 3a by the respective reference optical fibers 23.

The transmitted light guided into the light receiving unit 3a by the light receiving optical fibers 32 is incident on photodiodes 332a, 332b and 332c via, for example, a band-pass filter 331a for passing light with wavelengths in the vicinity of 575 nm which is absorbed to a large degree by clots of blood, a band-pass filter 331b for passing light with wavelengths in the vicinity of 650 nm which is absorbed to a different degree depending on the color of the egg shell and a band-pass filter 331c for passing light with wavelengths in the vicinity of 620 nm which is absorbed to a large degree by an addled egg, respectively. Each of the photodiodes 332a, 332b and 332c converts the incident light into an electrical signal according to the intensity of the light, and supplies the electrical signal to a comparing/amplifying unit 351.

On the other band, the transmitted light guided into the light receiving unit 3a by the reference optical fibers 23 is incident on photodiodes 342a, 342b and 342c after successively passing through ND filters (neutral-density filters) 341a, 341b and 341c as optical filters for limiting variations of the light quantity of the halogen lamp 241 and the above-mentioned band-pass filters 343a, 343b and 343c, respectively. Each of the photodiodes 342a, 342b and 342c converts the incident light into an electrical signal according to the intensity of the light, and supplies the electrical signal to the comparing/amplifying unit 351.

The comparing/amplifying unit 351 divides the electrical signals which have magnitudes corresponding to the intensity of the light transmitted through the egg E and are supplied from the photodiodes 332a, 332b and 332c by reference electrical signals which represent a state in which the egg E is not present and are supplied from the photodiodes 342a, 342b and 342c, respectively, amplifies the results of subtraction and supplies the amplified results to a gain selector 352. The gain selector 352 amplifies the three signals supplied from the comparing/amplifying unit 351 according to a preset gain, and supplies the resultant signals to the analog/digital (A/D) converter 36. The A/D converter 36 converts the three amplified analog signals supplied from the gain selector 352 into digital signals and supplies the resultant signals to a microcomputer 37a.

The microcomputer 37a performs normalizing processing for removing the effect of the egg shell by dividing a signal (first signal) corresponding to a wavelength of 575 nm among the three digital signals supplied from the A/D converter 36 by a signal (second signal) corresponding to a wavelength of 650 nm and dividing a signal (third signal) corresponding to a wavelength of 620 nm by the second signal so as to obtain first and third normalized signals.

In the microcomputer 37a, a first threshold value and a second threshold value used for judging whether the egg E is good or bad are preset. The microcomputer 37a judges whether the value of the first normalized signal is smaller than the first threshold value and whether the value of the third normalized signal is smaller than the second threshold value. If the value of either of the signals is smaller than the corresponding threshold value, the microcomputer 37a judges that the egg E is an abnormal egg and outputs a signal indicating this fact. On the other hand, if the values of both of the signals are equal to or larger than the respective threshold values, the microcomputer 37a judges that the egg E is a normal egg and outputs a signal indicating this fact.

Further, the microcomputer 37a corrects the gain of the gain selector 352 according to the result of judgement. Besides, the microcomputer 37a is connected to the above-mentioned connectors 46a and 46b, and transmits and receives data to/from the remote controller 6 via the connector 46a. In addition, the microcomputer 37a outputs the result of judgement to an external device of the egg inspecting device 1, for example, a selecting device of the eggs E via the other connector 46b.

This embodiment is configured as described above, and the same parts as in the first embodiment are designated by the same reference codes and the detailed explanation thereof are omitted.

### (Third Embodiment)

FIG. 12 is a side cross section showing the entire structure of the third embodiment of the egg inspecting device 1 according to the present invention from the right side. In contrast with the second embodiment, in the egg inspecting device 1 of this embodiment, the egg inspecting device main body 4 is separated from the light projecting end section 2 and the light receiving end section 3, and the light projecting end section and the light receiving end section 3 are formed as a single component. More specifically, the light projecting end section cover 20 and light receiving end section cover 30 possessed by the light projecting end section 2 and light receiving end section 3, respectively, in the second embodiment are connected together to form a light projecting and receiving end section cover 80 including a connecting portion 81 which has a suitable thickness from the bottom surface thereof and is laid over the transporting path 5, and this light projecting and receiving end section cover 80 is positioned separately from the main body housing 4.

According to such a structure, the light projecting optical fiber 22 and light receiving optical fibers 32 are led from suitable positions on the rear face of the main body housing 40, and led into the light projecting and receiving end section cover 80 from the front or rear elevation of the light projecting and receiving end section cover 80. Further, in the present invention, the configuration of leading the light projecting optical fiber 22 and light receiving optical fibers 23 is not limited to that mentioned above.

This embodiment is configured as described above, and other structures and functions are the same as those in the second embodiment. Therefore, the same parts are designated by the same reference codes and the detailed explanation thereof are omitted.

### (Fourth Embodiment)

FIG. 13 is a side cross section showing the entire structure of the fourth embodiment of the egg inspecting device 1 according to the present invention from the right side. In contrast with the second embodiment, in the egg inspecting device 1 of this embodiment, the egg inspecting device main body 4 is divided into two parts: the light projecting unit 2a and the light receiving unit 3a, and the light projecting end section 2 and the light receiving end section 3 are separated from the egg inspecting device main body 4 divided into two parts. More specifically, the light projecting end section cover 20 of the light projecting end section 2 and the light receiving end section cover 30 of the light receiving end section 3 of the second embodiment are respectively separated from the egg inspecting device main body 4, and the housing of the egg inspecting device main body 4 is divided into front and rear parts: a first main body housing 40a for storing the light projecting unit 2a and a second main body housing 40b for storing the light receiving unit 3a.

According to such a structure, the light projecting optical fiber 22 and reference optical fibers 23 are led from suitable positions on the rear elevation of the first main body housing 40a, the light projecting optical fiber 22 is led into the light projecting end section cover 20 from the front elevation, and the reference optical fibers 23 are led into the second main body housing 40b from the front elevation. Moreover, the light receiving optical fibers 32 are led from the front elevation of the second main body housing 40b, and led into the light receiving end section cover 30 from the rear elevation. Furthre, in the present invention, the configuration of leading the light projecting optical fiber 22, light receiving optical fibers 32 and reference optical fibers 23 is not limited to that mentioned above.

According to the above-described structure, the light receiving unit 3a having an electric part liable to be affected by heat is separated from a housing (the first main body housing 40a) for the halogen lamp 241 serving as a main heat source in the egg inspecting device 1, and disposed in another housing (the second main body housing 40b). It is thus possible to eliminate the necessity of the forced cooling system, for example, the cooling fan 45 as illustrated in the second embodiment.

This embodiment is configured as described above, and other structures and functions are the same as those in the second embodiment. Therefore, the same parts are designated by the same reference codes and the detailed explanation thereof are omitted.

### (Fifth Embodiment)

FIG. 14 is an external perspective view of the fifth embodiment of the egg inspecting device 1 according to the present invention, and FIG. 15 is a perspective view showing a state in which an egg is being inspected by the egg inspecting device 1 shown in FIG. 14. As shown in FIG. 15, in the transporting device 7 for transporting a plurality of eggs E, a plurality of arms 71 hanged from a plurality of supporting members (not shown) are arranged in an array at predetermined intervals. The lower end of each of the arms 71 is provided with a nip section 72 formed by attaching a plurality of fingers so that the fingers are freely movable in forward and backward directions, and the egg E is held by each nip section 72.

Each of the above-mentioned supporting members is supported by a horizontal rail (not shown) so that the supporting members can freely slide in forward and backward directions. By moving the respective supporting members with a drive device (not shown), the eggs E held by the respective nip sections 72 are transported in the direction shown by an arrow. The light projecting end section 2 for applying light to the egg E and the light receiving end section 3 on which the light transmitted through the egg E is incident are disposed to face each other in the egg inspecting device 1 to be described in detail below so that they face the transporting region of the eggs E.

The egg inspecting device 1 is provided with the above-described light projecting end section 2, light receiving end section 3, and egg inspecting device main body 4 including a later-described light source and a computing unit in a main body housing 40 in the shape of a rectangular paralellopiped shell. On the upper surface of the main body housing 40 of the egg inspecting device main body 4, the light projecting end section cover 20 and the light receiving end section cover 30 are disposed with a predetermined distance therebetween in a longitudinal direction of the upper surface of the main body housing 40. As described above, the eggs E pass through the transporting path 5 between the light projecting end section cover 20 and the light receiving end section cover 30.

Each of the light projecting end section cover 20 and light receiving end section cover 30 has substantially a right triangular shape when seen from side, and has a hexagonal shape formed by changing one side of a rectangle to a trapezoidal shape when seen from above. Moreover, the light projecting end section cover 20 and the light receiving end section cover 30 are positioned so that their upper bottom portions face each other. In other words, the center portions of the mutually facing surfaces of the light projecting end section cover 20 and light receiving end section cover 30 form parallel sections which are parallel with each other, and the portions on both sides of the center portions form tapered portions that flare with respect to each other toward both of the end portions. Therefore, when seen from above, the transporting path 5 has a so-called Japanese hand drum shape (bi-concave shape) with a narrower width at the center portion and a gradually increasing width toward both of the end portions. In the event of inspecting an egg E having a shorter diameter of up to about 50 mm by transporting the egg E, the width of the center portion of the above-described transport path 5 is about 64 mm.

FIG. 16 is a block diagram showing the structure of the egg inspecting device main body 4 shown in FIG. 15. The egg inspecting device main body 4 incorporates a light source 24 including the halogen lamp 241, reflector 242 and heat ray cut filter 243 for cutting a heat ray (infrared ray) in a housing 24a, and an optical fiber 21 is connected to the housing 24a. Light emitted from the halogen lamp 241 is incident on the heat ray cut filter 243 directly or after being reflected by the reflector 242. The heat ray is cut by the heat ray cut filter 243, and the resultant light is incident on the optical fiber 21.

A plurality of strands of the optical fiber 21 with a low numerical aperture (NA of about 0.56) is branched into one thick bundle and three mutually identical thin bundles. The thick bundle serving as the light projecting optical fiber 22 is passed through a through-hole 48a made through the upper surface of the main body housing 40 and is extended into the light projecting end section cover 20, while the thin bundles serving as the reference optical fibers 23 are extended to a substrate 31 on which the later-described amplifier 35, computing unit 37, etc. are mounted. The light emitting end of the light projecting optical fiber 22 is fitted and fastened in a light projecting cylinder 220 which has a circular shape in its front view and is formed from a light blocking material. A light projection hole 29 is formed to pierce the light projecting end section cover 20 at substantially the center of a surface of the light projecting end section cover 20, which surface faces the transport path 5. The light projecting cylinder 220 is positioned so that its central axis is aligned with the central axis of the light projection hole 29.

Moreover, a light reception hole 39 is formed at a portion of the light receiving end section cover 30, which portion faces the above-described light projection hole 29. A light receiving cylinder 320, which is formed from a light blocking material and in which the light receiving optical fiber 32 is fitted and fastened, is positioned so that its central axis is aligned with the central axis of the light reception hole 39.

FIG. 17 is a side cross section of the light projecting cylinder 220 and the vicinity of the light emitting end of the light projecting optical fiber 22 shown in FIG. 16. The internal diameter of the light projecting cylinder 220 is made smaller in the center portion and made larger in both of the end portions, and a dimension at the center portion in the axial direction is determined according to a focal length of a later-described condenser lens 221. A light projecting end 22a of the light projecting optical fiber 22 is fitted into one end of the light projecting cylinder 220, and fastened with a screw 223 or the like. The condenser lens 221 such as a collimate lens is installed in the other end of the light projecting cylinder 220, and fastened with a ring-shaped press member 222 screwed to the other end of the light projecting cylinder 220.

The light guided by the light projecting optical fiber 22 and emitted from the light projecting end 22a is incident on the condenser lens 221 where the light is condensed into a light beam of a predetermined diameter and then emitted from the other end of the light projecting cylinder 220. This light is applied to the egg E. The internal diameter of the center portion of the light projecting cylinder 220 is approximately 13 mm, and the light condensed by the condenser lens 221 is emitted from the light projecting cylinder 220. Therefore, substantially the whole emitted light is applied to the egg E. Hence, a rate at which the light emitted from the light projecting end section 2 becomes stray light is reduced, and the utilization factor of the emitted light is improved.

FIG. 18 is a side cross section of the light receiving cylinder 320 and the vicinity of the light incident end of the light receiving optical fiber 32 shown in FIG. 16. The light receiving end 32a of the light receiving optical fiber 32 is fitted into one end portion of the light receiving cylinder 320 having a circular shape in its front view, and fastened to the light receiving cylinder 320 with a screw 322. The internal diameter of the center portion of the light receiving cylinder 320 is made smaller than that of the above-mentioned one end portion, while the internal diameter of the other end portion of the light receiving cylinder 320 is made smaller than that of the center portion. A protecting member 321 formed by providing a transparent cover 321b in a ring-shaped supporting member 321a is fitted and screwed onto the other end of the light receiving cylinder 320 so as to prevent the entry of foreign matter into the light receiving cylinder 320.

The light receiving cylinder 320 is positioned so that its central axis is aligned with the central axis of the above-described light projecting cylinder 220. The light emitted from the light projecting cylinder 220 is incident on the light receiving cylinder 320, while other light scarcely enters the light receiving cylinder 320, thereby improving the egg inspecting accuracy.

Further, although this embodiment illustrates an example in which the condenser lens 221 is provided in the above-described light projecting cylinder 220, the present invention is not necessarily limited to this structure and may not have the condenser lens 221 in the light projecting cylinder 220. In this case, the light projecting end 22a of the light projecting optical fiber 22 is positioned in the light projecting cylinder 220, and light diverging from the light projecting end 22a is blocked by the light projecting cylinder 220. It is therefore possible to prevent divergence of the light emitted from the light projecting end section 2 irrespective of the numerical aperture of the light projecting optical fiber 22.

The light incident on the light receiving cylinder 320 is guided into the light receiving optical fiber 32. As shown in FIG. 16, for the light receiving optical fiber 32, three branch fibers formed by three bundles of the same number of strands which are the same as that of the above-described optical fiber 21 are used. Each branch fiber is passed through a through-hole 48b made through the upper surface of the main body housing 40 and is extended to the substrate 31. Six sensor blocks 33 each of which incorporates a later-described photoelectric converter are mounted on the substrate 31. The light emitting ends of the branched light receiving optical fibers 32 are connected to three sensor blocks 33 among the six sensor blocks 33, respectively. Besides, the light emitting ends of the above-described reference optical fibers 23 are connected to the other three sensor blocks 33, respectively.

FIG. 19 is a side cross section of the sensor block 33 shown in FIG. 16 and a light emitting end 33a connected to this sensor block 33. A disk-shaped photoelectric converter 334 is fitted into one end of the cylindrical sensor block 33, while the light emitting end 33a of any one of the above-described fibers is put into the other end of the sensor block 33. A band-pass filter 333 made of an interference filter is provided on the input side of the photoelectric converter 334, and a ball lens 331 as a collimate lens is disposed between the band-pass filter 333 and the light emitting end 33a. The ball lens 331 is held between a pair of spacers 332. A dimension between the ball lens 331 and the light emitting end 33a and a dimension between the ball lens 331 and the light receiving surface of the photoelectric converter 334 are adjusted to predetermined dimensions by the spacers 332.

The light emitted from the light emitting end 33a is incident on the ball lens 331 where it is made parallel light and incident on the band-pass filter 333, and then light in a required wavelength band is emitted from the band-pass filter 333 to the photoelectric converter 334. Then, the photoelectric converter 334 outputs an electrical signal corresponding to the intensity of the incident light.

Since the light which has been made parallel light by the ball lens 331 is incident on the band-pass filter 333, it is possible to prevent a deviation of wavelength selected by the band-pass filter 333.

The three sensor blocks 333 to which the branched light receiving optical fibers 32 are connected are provided with, for example, a band-pass filter for passing light with wavelengths in the vicinity of 575 nm which is absorbed to a large degree by clots of blood, a band-pass filter for passing light with wavelengths in the vicinity of 650 nm which is absorbed to a different degree depending on the color of the egg shell, and a band-pass filter for passing light with wavelengths in the vicinity of 620 nm which is absorbed to a large degree by an addled egg, respectively. In order to form pairs with these sensor blocks 33, three sensor blocks 33 to which the reference optical fibers 23 are connected are provided with a band-pass filter for passing light with wavelengths in the vicinity of 575 nm, a band-pass filter for passing light with wavelengths in the vicinity of 650 nm and a band-pass filter for passing light with wavelengths in the vicinity of 620 nm, respectively.

Signals output from the respective photoelectric converters provided in the sensor blocks 33 are supplied to the amplifier 35, and the resultant amplified signals are supplied to the computing unit 37 via the analog/digital (A/D) converter 36. As described above, the wavelength bands of the band-pass filters provided for three sensor blocks 33 to which the branched light receiving optical fibers 32 are connected are arranged to correspond to the wavelength bands of the band-pass filters provided for three sensor blocks 33 to which the reference optical fibers 23 are connected. The computing unit 37 is supplied via the A/D) converter 36 with a first inspection signal (signal related to wavelengths in the vicinity of 575 nm of the light transmitted through an egg), a second inspection signal (signal related to wavelengths in the vicinity of 650 nm of the light transmitted through an egg) and a third inspection signal (signal related to wavelengths in the vicinity of 620 nm of the light transmitted through an egg) from three sensor blocks 33 to which the light receiving optical fibers 32 are connected, and is also supplied with a first reference signal (signal related to wavelengths in the vicinity of 575 nm of the light source light), a second reference signal (signal related to wavelengths in the vicinity of 650 nm of the light source light) and a third reference signal (signal related to wavelengths in the vicinity of 620 nm of the light source light) corresponding to the first to third inspection signals, from three sensor blocks 33 to which the reference optical fibers 23 are connected.

The computing unit 37 obtains first to third correction signals by dividing the first to third inspection signals by the corresponding first to third reference signals, and performs normalizing processing for removing the effect of the color of the egg shell from the first and third correction signals by dividing the first correction signal by the second correction signal and dividing the third correction signal by the second correction signal so as to obtain a first normalized signal and a third normalized signal. In the computing unit 37, first and second threshold values used for judging whether the egg E is good or bad are preset. The computing unit 37 judges whether the values of the first and third normalized signals are greater than the first and second threshold values, respectively and judges that the egg E is an abnormal egg if the value of either of the signals is greater than the threshold value and outputs a signal indicating this fact, or judges that the egg E is a normal egg if the values of both of the signals are not greater than the threshold values and outputs a signal indicating this fact.

Further, although this embodiment includes the band-pass filters of predetermined wavelength bands to detect abnormal eggs such as eggs containing clots of blood and addled eggs, the present invention does not necessarily include such band-pass filters. Needless to say, band-pass filters for transmitting light in a wavelength band corresponding to other internal abnormality, such as eggs containing meat-like substances, may be provided.

Besides, although reference codes are added in the claims for the sake of convenience in referring to drawings, they are not intended to limit the present invention to the structures shown in the accompanying drawings.

### INDUSTRICAL APPLICABILITY

As described above, the present invention are able to detect very small internal abnormality and inspect whether eggs are good or bad with high accuracy, irrespective of the types of the egg shell and the sizes of the eggs.

Besides, in an egg inspecting device which applies light from a light source such as a halogen lamp by guiding it by a first optical system, guides the light which has been applied to and transmitted through the egg to a photoelectric converter by a second optical system, performs photoelectric conversion of the guided light in a photoelectric converter and inspects the inside of the egg by an inspecting section based on the magnitude of a resultant electrical signal, since the first and second optical systems are partially or entirely formed by optical fibers, this egg inspecting device can be readily introduced into an egg transporting system without being restricted by the size of a light projecting unit including the light source and first optical system and the size of a light receiving unit including the second optical system, photoelectric converter and inspecting section, and the degree of freedom in disposing both of the units can be increased.

Furthermore, since the light emitting end of the first optical system to the egg and the light incident end of the second optical system from the egg are positioned in another housing separated from a housing storing the light source, photoelectric converter and inspecting section, it is possible to have the above-mentioned light emitting end and light incident end separately from most parts of the device by the optical fibers, thereby further improving the degree of freedom in disposing the device. In addition, since the above-mentioned light emitting end and light incident end are positioned in a single housing, it is possible to eliminate the necessity of aligning the optical axes of these light emitting end and light incident end.

Besides, the light emitting end of the first optical system to the egg is positioned in a second housing which is separated from a first housing storing the light source, and the light incident end of the second optical system from the egg is positioned in a fourth housing which is separated from a third housing storing the photoelectric converter and inspecting section. With such a configuration, it is possible to have the light projecting unit and the light receiving unit separately, thereby further improving the degree of freedom in disposing the device. Moreover, since the light emitting end of the first optical system is positioned in a housing different from a housing of the main body section of the light projecting unit and the light incident end of the second optical system is positioned in a housing different from a housing of the main body section of the light receiving unit, the degree of freedom in disposing the device is further improved.

Furthermore, since the ratio of the light applied to the egg to the light emitted by the projector is improved irrespective of the numerical aperture of a first light guiding body, the quantity of light transmitted through the egg is increased, while the stray light is reduced, thereby improving the detection accuracy of abnormal eggs.

Additionally, in the case where a light emitting-side end portion of the first light guiding body is positioned in a light blocking cylinder provided in the projector, even when the numerical aperture of the first light guiding body is relatively large, the cylinder prevents divergence of the emitted light from the projector, thereby reducing stray light which is not applied to the egg. Besides, in the case where a light incident-side end portion of a second light guiding body is positioned in a light blocking cylinder provided in a light receiver, light which has been transmitted through an egg and travels substantially parallel to the axial direction of the cylinder enters the cylinder, while external light and stray light which travel in a direction across the axial direction of the cylinder can hardly enter the cylinder. Therefore, even when the numerical aperture of the second light guiding body is relatively large, it is possible to prevent the external light and stray light from falling on the second light guiding body. Hence, the detection accuracy of abnormal eggs is improved.

Furthermore, since it is possible to prevent a deviation of the wavelength of light passing through an interference filter on which the light transmitted through the egg is incident by the guide of the second light guiding body, the reliability of the detection of abnormal eggs is high.

Additionally, since another collimate lens is installed between another interference filter and a light emitting-side end portion of a third light guiding body for guiding the light emitted by the light source to a detector so as to obtain a reference, it is possible to prevent a deviation of the wavelength of light passing through the another interference filter and provide a highly reliable reference. The present invention produces such advantageous effects.

## Claims

1. An egg inspecting method comprising applying light including predetermined wavelengths to an egg (E) from a light source (24, 241) so as to obtain transmitted light; measuring an intensity of a first wavelength which is absorbed to a large degree by internal abnormality of the egg (E) and an intensity of a second wavelength which is absorbed to a different degree depending on a type of a shell of the egg (E) respectively by using the transmitted light; and detecting internal abnormality of the egg (E) based on the measured intensities of the first and second wavelengths,
said egg inspecting method being characterized by measuring an intensity of a third wavelength which is absorbed to a small degree by the internal abnormality by using the transmitted light, normalizing the measured intensities of the third wavelength and first wavelength respectively by the intensity of the second wavelength, calculating a difference between the normalized intensity of the first wavelength and the normalized intensity of the third wavelength, and judging whether the egg (E) has internal abnormality based on the calculated difference.

2. An egg inspecting device comprising a measuring section (36, 331a, 332a, 333, 334a, 335a) for measuring an intensity of a first wavelength which is included in transmitted light obtained by applying light including predetermined wavelengths to an egg (E) and absorbed to a large degree by internal abnormality of the egg (E); a measuring section (36, 331b, 332b, 333, 334b, 335b) for measuring an intensity of a second wavelength which is absorbed to a different degree depending on a type of a shell of the egg (E) by using the transmitted light; and a detector (37, 37a) for detecting internal abnormality of the egg (E) based on the measured intensities of the first and second wavelengths,
said egg inspecting device being characterized by comprising a measuring section (36, 331c, 332c, 333, 334c, 335c) for measuring an intensity of a third wavelength which is absorbed to a small degree by the internal abnormality by using the transmitted light, and characterized in that said detector (37, 37a) includes a normalizing section (371) for normalizing the third intensity and the first intensity respectively by the second intensity, and a difference calculating section (372) for calculating a difference between the normalized first intensity and the normalized third intensity.

3. The egg inspecting device as set forth in claim 2, further comprising: a light source (24, 241); a first optical system (22, 242, 243) for guiding the light from said light source (24, 241) so as to be applied to the egg (E); and a second optical system (32) for guiding the transmitted light from the egg (E) to said respective measuring sections (36, 331a to 331c, 332a to 332c, 333, 334a to 334c, 335a to 335c), wherein said first optical system (22, 242, 243) and said second optical system (32) are partially or entirely formed by optical fibers (22, 32).

4. The egg inspecting device as set forth in claim 3, wherein a light emitting end (22a) of said first optical system (22, 242, 243) to the egg (E) and a light incident end (32a) of said second optical system (32) from the egg (E) are disposed in another housing (80) separated from a housing (40) storing said light source (24, 241), measuring sections (36, 331a to 331c, 332a to 332c, 333, 334a to 334c, 335a to 335c) and detector (37, 37a).

5. The egg inspecting device as set forth in claim 3, wherein a light emitting end (22a) of said first optical system (22, 242, 243) to the egg (E) is disposed in a second housing (20) separated from a first housing (40a) storing said light source (24, 241), and a light incident end (32a) of said second optical system (32) from the egg (E) is disposed in a fourth housing (30) separated from a third housing (40b) storing said measuring sections (36, 331a to 331c, 332a to 332c, 333, 334a to 334c, 335a to 335c) and detector (37, 37a).

6. The egg inspecting device as set forth in claim 2, further comprising: a first light guiding body (22) for guiding light emitted by said light source (24, 241); a projector (2) for emitting the light guided by said first light guiding body (22) to the egg (E); a light receiver (3) on which the transmitted light through the egg (F) is incident; and a second light guiding body (32) for guiding the transmitted light incident on said light receiver (3) to said respective measuring sections (36, 331a to 331c, 332a to 332c, 333, 334a to 334c, 335a to 335c), wherein said projector (2) includes a condenser lens (221) for condensing the light emitted from said first light guiding body (22).

7. The egg inspecting device as set forth in claim 2, further comprising: a first light guiding body (22) for guiding light emitted by said light source (24, 241); a projector (2) for emitting the light guided by said first light guiding body (22) to the egg (E); a light receiver (3) on which the transmitted light through the egg (E) is incident; and a second light guiding body (32) for guiding the transmitted light incident on said light receiver (3) to said respective measuring sections (36, 331a to 331c, 332a to 332c, 333, 334a to 334c, 335a to 335c), wherein said projector (2) and/or said light receiver (3) include(s) a light blocking cylinder (220, 320), and said first light guiding body (22) and/or said second light guiding body (32) are/is connected to said cylinder(s) (220, 320) in such a manner that a light emitting-side end portion (22a) of said first light guiding body (22) and/or a light incident-side end portion (32a) of said second light guiding body (32) are/is positioned in said cylinder(s) (220, 320).

8. The egg inspecting device as set forth in claim 2, further comprising: a first light guiding body (22) for guiding light emitted by said light source (24, 241); a projector (2) for emitting the light guided by said first light guiding body (22) to the egg (E); a light receiver (3) on which the transmitted light through the egg (E) is incident; and a second light guiding body (32) for guiding the transmitted light incident on said light receiver (3) to said respective measuring sections (36, 331a to 331c, 332a to 332c, 333, 334a to 334c, 335a to 335c), wherein an interference filter (331a to 331c, 333) for passing light in a predetermined wavelength band is disposed to face a light emitting-side end portion (33a) of said second light guiding body (32), and a collimate lens (331) is installed between the light emitting-side end portion (33a) of said second light guiding body (32) and said interference filter (331a to 331c, 333).

9. The egg inspecting device as set forth in claim 8, further comprising a third light guiding body (23) for guiding light emitted by said light source (24, 241) to said respective measuring sections (36, 334a to 334c, 335a to 335c), wherein another interference filter (333, 343a to 343c) for passing light in the same wavelength band as the wavelength band of said interference filter (331a to 331c, 333) is disposed to face a light emitting-side end portion (33a) of said third light guiding body (23), and another collimate lens (331) is installed between the light emitting-side end portion (33a) of said third light guiding body (23) and said another interference filter (333, 343a to 343c).

10. An egg inspecting device comprising a light source (24, 241); a first optical system (22, 221, 242, 243) for guiding light from said light source (24, 241) so as to be applied to an egg (E); a second optical system (32, 331a to 331c, 333) for guiding transmitted light through the egg (E); photoelectric converters (332a to 332c, 334, 334a to 334c) for performing photoelectric conversion of the light guided by said second optical system (32, 331a to 331c, 333); and an inspecting section (37, 37a) for inspecting inside of the egg (E) based on magnitudes of electrical signals obtained from said photoelectric converters (332a to 332c, 334, 334a to 334c),
said egg inspecting device being characterized in that said first optical system (22, 221, 242, 243) and said second optical system (32, 331a to 331c, 333) are partially or entirely formed by optical fibers (22, 32).

11. The egg inspecting device as set forth in claim 10, wherein a light emitting end (22a) of said first optical system (22, 221, 242, 243) to the egg (E) and a light incident end (32a) of said second optical system (32, 331a to 331c, 333) from the egg (E) are disposed in another housing (80) separated from a housing (40) storing said light source (24, 241), photoelectric converters (332a to 332c, 334, 334a to 334c) and inspecting section (37, 37a).

12. The egg inspecting device as set forth in claim 10, wherein a light emitting end (22a) of said first optical system (22, 221, 242, 243) to the egg (E) is disposed in a second housing (20) separated from a first housing (40a) storing said light source (24, 241), and a light incident end (32a) of said second optical system (32, 331a to 331c, 333) from the egg (E) is disposed in a fourth housing (30) separated from a third housing (40b) storing said photoelectric converters (332a to 332c, 334, 334a to 334c) and inspecting section (37, 37a).

13. The egg inspecting device as set forth in any one of claims 10 through 12, wherein said second optical system (32, 331a to 331c, 333) further comprises a first optical component (331a, 333) for transmitting light with a first wavelength which is included in transmitted light obtained by applying light including predetermined wavelengths to the egg (E) and absorbed to a large degree by internal abnormality of the egg (E); a second optical component (331b, 333) for transmitting light with a second wavelength which is absorbed to a different degree depending on a type of a shell of the egg (E) by using the transmitted light; and a third optical component (331c, 333) for transmitting light with a third wavelength which is absorbed to a small degree by the internal abnormality by using the transmitted light, and said inspecting section (37, 37a) includes a normalizing section (371) for normalizing a third electrical signal derived from transmitted light from said third optical component (331c, 333) and a first electrical signal derived from transmitted light from said first optical component(331a, 333) respectively by a second electrical signal derived from transmitted light from said second optical component (331b, 333); and a difference calculating section (372) for calculating a difference between the normalized first and third electrical signals.

14. The egg inspecting device as set forth in any one of claims 10 through 13, wherein said first optical system (22, 221, 242, 243) includes a condenser lens (221) for condensing light emitted from said optical fibers (22, 32).

15. The egg inspecting device as set forth in any one of claims 10 through 13, wherein said first optical system (22, 221, 242, 243) and/or said second optical system (32, 331a to 331c, 333) include(s) a light blocking cylinder (220, 320), and the optical fibers (22, 32) of said first optical system (22, 221, 242, 243) and/or the optical fibers (22, 32) of said second optical system (32, 331a to 331c, 333) are connected to said cylinder(s) (220, 320) in such a manner that the light emitting-side end portion (22a) of the optical fibers (22, 32) of said first optical system (22, 221, 242, 243) and/or the light incident-side end portion (32a) of the optical fibers (22, 32)of said second optical system (32, 331a to 331c, 333) are/is positioned in said cylinder(s) (220, 320).

16. The egg inspecting device as set forth in any one of claims 10 through 13, wherein said second optical system (32, 331a to 331c, 333) comprises an interference filter (331a to 331c, 333), disposed to face a light emitting-side end portion (33a) of said optical fiber (22, 32), for passing light in a predetermined wavelength band; and a collimate lens (331) installed between the light emitting-side end portion (33a) of said optical fiber (22, 32) and said interference filter (331a to 331c, 333).

17. The egg inspecting device as set forth in claim 16, further comprising an optical fiber (23) for guiding light emitted by said light source (24, 241) to said inspecting section (37, 37a) directly, wherein another interference filter (333, 343a to 343c) for passing light in the same wavelength band as the wavelength band of said interference filter (331a to 331c, 333) is disposed to face a light emitting-side end portion (33a) of said optical fiber (23), and another collimate lens (331) is installed between the light emitting-side end portion (33a) of said optical fiber (23) and said another interference filter (333, 343a to 343c).

18. An egg inspecting device comprising a light source (24, 241); a first light guiding body (22) for guiding light emitted by said light source (24, 241); a projector (2) for emitting the light guided by said first light guiding body (22) to an egg (E); a light receiver (3) on which transmitted light through the egg (E) is incident; a second light guiding body (32) for guiding the transmitted light incident on said light receiver (3); and a detector (37, 37a) for detecting internal abnormality of the egg (E) based on an intensity of the transmitted light guided by said second light guiding body (32),
wherein said projector (2) includes a condenser lens (221) for condensing the light emitted from said first light guiding body (22).

19. The egg inspecting device as set forth in claim 18, wherein said second light guiding body (32) further comprises a first optical component (101) for transmitting light with a first wavelength which is included in transmitted light obtained by applying light including predetermined wavelengths to the egg (E) and absorbed to a large degree by internal abnormality of the egg (E); a second optical component (102) for transmitting light with a second wavelength which is absorbed to a different degree depending on a type of a shell of the egg (E) by using the transmitted light; and a third optical component (103) for transmitting light with a third wavelength which is absorbed to a small degree by the internal abnormality by using the transmitted light, and said detector (37, 37a) includes a normalizing section (371) for normalizing a third electrical signal derived from transmitted light from said third optical component (103) and a first electrical signal derived from transmitted light from said first optical component (101) respectively by a second electrical signal derived from transmitted light from said second optical component (102); and a difference calculating section (372) for calculating a difference between the normalized first and third electrical signals.

20. The egg inspecting device as set forth in either of claims 18 and 19, wherein said first light guiding body (22) and said second light guiding body (32) are partially or entirely formed by optical fibers (22, 32).

21. The egg inspecting device as set forth in any one of claims 18 through 20, wherein said projector (2) and light receiver (3) are disposed in another housing (80) separated from a housing (40) storing said light source (24, 241) and detector (37, 37a).

22. The egg inspecting device as set forth in any one of claims 18 through 20, wherein said projector (2) is disposed in a second housing (20) separated from a first housing (40a) storing said light source (24, 241), and said light receiver (3) is disposed in a fourth housing (30) separated from a third housing (40b) storing said detector (37, 37a).

23. An egg inspecting device comprising a light source (24, 241); a first light guiding body (22) for guiding light emitted by said light source (24, 241); a projector (2) for emitting the light guided by said first light guiding body (22) to an egg (E); a light receiver (3) on which transmitted light through the egg (E) is incident; a second light guiding body (32) for guiding the transmitted light incident on said light receiver (3); and a detector (37, 37a) for detecting internal abnormality of the egg (E) based on an intensity of the transmitted light guided by said second light guiding body (32),
wherein said projector (2) and/or said light receiver (3) include(s) a light blocking cylinder (220, 320), and said first light guiding body (22) and/or said second light guiding body (32) are connected to said cylinder(s) (220, 320) in such a manner that a light emitting-side end portion (22a) of said first light guiding body (22) and/or a light incident-side end portion (32a) of said second light guiding body (32) are/is positioned in said cylinder(s) (220, 320).

24. The egg inspecting device as set forth in claim 23, wherein said second light guiding body (32) further comprises a first optical component (331a, 333) for transmitting light with a first wavelength which is included in transmitted light obtained by applying light including predetermined wavelengths to the egg (E) and absorbed to a large degree by internal abnormality of the egg (E); a second optical component (331b, 333) for transmitting light with a second wavelength which is absorbed to a different degree depending on a type of a shell of the egg (E) by using the transmitted light; and a third optical component (331c, 333) for transmitting light with a third wavelength which is absorbed to a small degree by the internal abnormality by using the transmitted light, and said detector (37, 37a) includes a normalizing section (371) for normalizing a third electrical signal derived from transmitted light from said third optical component (331c, 333) and a first electrical signal derived from transmitted light from said first optical component(331a, 333) respectively by a second electrical signal derived from transmitted light from said second optical component (331b, 333); and a difference calculating section (372) for calculating a difference between the normalized first and third electrical signals.

25. The egg inspecting device as set forth in claim 23 or 24, wherein said first light guiding body (22) and said second light guiding body (32) are partially or entirely formed by optical fibers (22, 32).

26. The egg inspecting device as set forth in any one of claims 23 through 25, wherein said projector (2) and light receiver (3) are disposed in another housing (80) separated from a housing (40) storing said light source (24, 241) and detector (37, 37a).

27. The egg inspecting device as set forth in any one of claims 23 through 25, wherein said projector (2) is disposed in a second housing (20) separated from a first housing (40a) storing said light source (24, 241), and said light receiver (3) is disposed in a fourth housing (30) separated from a third housing (40b) storing said detector (37, 37a).

28. An egg inspecting device comprising a light source (24, 241); a first light guiding body (22) for guiding light emitted by said light source (24, 241); a projector (2) for emitting the light guided by said first light guiding body (22) to an egg (E); a light receiver (3) on which transmitted light through the egg (E) is incident; a second light guiding body (32) for guiding the transmitted light incident on said light receiver (3); and a detector (37, 37a) for detecting internal abnormality of the egg (E) based on an intensity of the transmitted light guided by said second light guiding body (32),
wherein an interference filter (331a to 331c, 333) for transmitting light in a predetermined wavelength band is disposed as said detector (37, 37a) to face a light emitting-side end portion (22a) of said second light guiding body (32), and a collimate lens (331) is installed between the light emitting-side end portion (22a) of said second light guiding body (32) and said interference filter (331a to 331c, 333).

29. The egg inspecting device as set forth in claim 28, further comprising a third light guiding body (23) for guiding light emitted by said light source (24, 241) to said detector (37, 37a), wherein another interference filter (333) for passing light in the same wavelength band as the wavelength band of said interference filter (331a to 331c, 333) is disposed to face a light emitting-side end portion (33a) of said third light guiding body (23), and another collimate lens (331) is installed between the light emitting-side end portion (22a) of said third light guiding body (23) and said another interference filter (333).

30. The egg inspecting device as set forth in claim 28 or 29, wherein said second light guiding body (32) further comprises a first optical component (331a) for transmitting light with a first wavelength which is included in transmitted light obtained by applying light including predetermined wavelengths to the egg (E) and absorbed to a large degree by internal abnormality of the egg (E); a second optical component (331b) for transmitting light with a second wavelength which is absorbed to a different degree depending on a type of a shell of the egg (E) by using the transmitted light; and a third optical component (331c) for transmitting light with a third wavelength which is absorbed to a small degree by the internal abnormality by using the transmitted light, and said detector (37, 37a) includes a normalizing section (371) for normalizing a third electrical signal derived from transmitted light from said third optical component (103) and a first electrical signal derived from transmitted light from said first optical component (101) respectively by a second electrical signal derived from transmitted light from said second optical component (102); and a difference calculating section (372) for calculating a difference between the normalized first and third electrical signals.

31. The egg inspecting device as set forth in any one of claims 28 through 30, wherein said first light guiding body (22) and said second light guiding body (32) are partially or entirely formed by optical fibers (22, 32).

32. The egg inspecting device as set forth in any one of claims 28 through 31, wherein said projector (2) and light receiver (3) are disposed in another housing (80) separated from a housing (40) storing said light source (24, 241) and detector (37, 37a).

33. The egg inspecting device as set forth in any one of claims 28 through 31, wherein said projector (2) is disposed in a second housing (20) separated from a first housing (40a) storing said light source (24, 241), and said light receiver (3) is disposed in a fourth housing (30) separated from a third housing (40b) storing said detector (37, 37a).
